(19) Europäisches Patentamt
European Patent Office
Office européen des brevets

(11) Numéro de publication : **0 487 423 A1**

(12) # DEMANDE DE BREVET EUROPEEN

(21) Numéro de dépôt : **91403146.3**

(22) Date de dépôt : **22.11.91**

(51) Int. Cl.[5] : **C07D 293/12,** C07C 391/02, A61K 31/41, A61K 31/095, C07D 421/06

(30) Priorité : **23.11.90 FR 9014591**

(43) Date de publication de la demande :
**27.05.92 Bulletin 92/22**

(84) Etats contractants désignés :
**AT BE CH DE DK ES FR GB GR IT LI LU NL SE**

(71) Demandeur : **ADIR ET COMPAGNIE**
**1 rue Carle Hébert**
**F-92415 Courbevoie Cédex (FR)**

(72) Inventeur : **Galet, Vincent**
**28 rue Berlioz**
**F-59210 Coudekerque Branche (FR)**

Inventeur : **Vaccher, Marie-Pierre**
**27 rue du Trianon**
**F-59139 Wattignies (FR)**
Inventeur : **Lesieur, Daniel**
**20 rue de Verdun**
**F-59147 Gondecourt (FR)**
Inventeur : **Renard, Pierre**
**50 avenue de Villeneuve l'Etang**
**F-78000 Versailles (FR)**
Inventeur : **Caignard, Daniel-Henri**
**69 bis rue Brancion**
**F-75015 Paris (FR)**
Inventeur : **Renaud de la Faverie, Jean-François**
**7 rue des Erables - Rocquencourt**
**F-78150 Le Chesnay (FR)**
Inventeur : **Adam, Gérard**
**9 clos du Mesnil - route du Pecq**
**F-78600 Le Mesnil le Roi (FR)**

(54) **Nouveaux dérivés de la benzosélénazolinone, leur procédé de préparation et les compositions pharmaceutiques qui lescontiennent.**

(57) Composés de formule générale (I) :

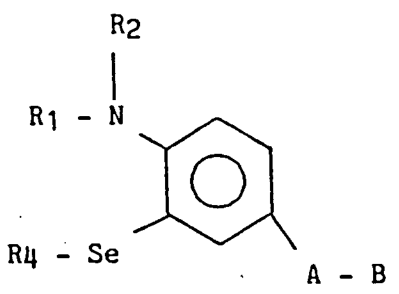

(I)

dans laquelle :

— $R_1$ représente soit un atome d'hydrogène soit un groupement $CO-NR_7R_8$ avec $R_7$, $R_8$ identiques ou différents, réprésentant un atome d'hydrogène, un groupement alkyle inférieur ou cycloalkyle de 3 à 8 atomes de carbone ou aryle, ou aryle substitué, ou arylalkyle inférieur, ou arylalkyle inférieur substitué ou forment avec l'azote qui les porte un système hétérocyclique,

— $R_4$ représente un groupement :

EP 0 487 423 A1

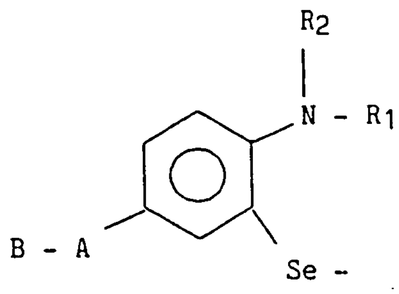

ou bien :

$R_4$ forme avec $R_1$ un groupement -C = O,

— $R_2$ représente un atome d'hydrogène ou un groupement alkyle inférieur éventuellement substitué par un ou plusieurs atomes d'halogène ou par un groupement hydroxy, ou lorsque $R_1$ forme avec $R_4$ un groupement CO, par un groupement $NR_5R_6$, dans laquelle $R_5$ et $R_6$, identiques ou différents représentent chacun indépendamment l'un de l'autre un atome d'hydrogène, un groupement alkyle inférieur, un groupement aryle, un groupement aryle substitué, un groupement arylalkyle inférieur, un groupement arylalkyle inférieur substitué,

ou bien $R_5$ et $R_5$ forment avec l'atome d'azote qui les porte un système hétérocyclique,

— A représente un groupement CO, CHOH ou $CH_2$,

— B représente un atome d'hydrogène, un groupement alkyle inférieur, un groupement cycloalkyle de 3 à 8 atomes de carbone, un groupement alcényle inférieur, un groupement alcynyle inférieur, un groupement aryle ou un groupement aryle substitué, un groupement hétéroaryle ou un groupement hétéroaryle substitué, un groupement arylalkyle inférieur, un groupement hétéroarylalkyle inférieur ou un groupement hétéroarylalkyle inférieur substitué, un groupement styryle ou un groupement styryle substitué,

ou A et B représentent ensemble un atome d'hydrogène à la condition que, lorsque A et B forment ensemble un atome d'hydrogène

. si $R_1$ forme avec $R_4$ un groupement CO alors $R_2$ ne peut représenter ni groupement méthyle, ni un atome d'hydrogène,

. si $R_1$ ne forme par avec $R_4$ un groupement CO alors $R_1$ et $R_2$ ne peuvent simultanément représenter un atome d'hydrogèbe

Médicaments.

La présente invention concerne de nouveaux dérivés de la benzoselenazolinone, leur procédé de préparation et les compositions pharmaceutiques qui les contiennent.

La demande de brevet européen 0 044 971 décrit l'activité antirhumatismale de la 2-phényl-1,2-benzisoselenazol-3[2H]one. La présente invention a découvert de nouveaux dérivés de la benzo [2H] selenazol-3 one, ou benzoselenazolinone, dont l'activité sur divers tests révélateurs d'une activité antioxydante est nettement supérieure à celle du dérivé dont il est fait mention dans la demande de brevet EP 0 044 971. Ce meilleur niveau d'activité, lié à la faible toxicité des dérivés de l'invention, permet l'administration de doses plus faibles, ce qui est particulièrement intéressant compte tenu du caractère chronique du traitement des maladies pour lesquelles les dérivés de l'invention sont susceptibles d'être administrés. En outre les dérivés de l'invention ont montré de bonnes propriétés antiagrégantes plaquettaires et immunostimulantes qui les rendent intéressants dans des indications thérapeutiques différentes de celles indiquées dans la demande EP 0 044 971.

Plus spécifiquement, l'invention concerne des dérivés de formule générale (I) :

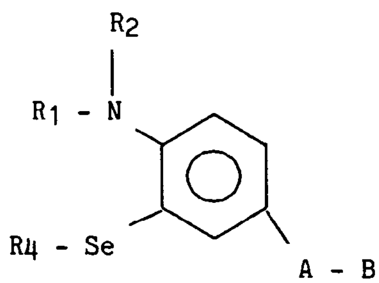

(I)

dans laquelle :

– $R_1$ représente soit un atome d'hydrogène soit un groupement $CO-NR_7R_8$ avec $R_7$, $R_8$ identiques ou différents, réprésentant un atome d'hydrogène, un groupement alkyle inférieur ou cycloalkyle de 3 à 8 atomes de carbone ou aryle, ou aryle substitué, ou arylalkyle inférieur, ou arylalkyle inférieur substitué ou forment avec l'azote qui les porte un système hétérocyclique,

– $R_4$ représente un groupement :

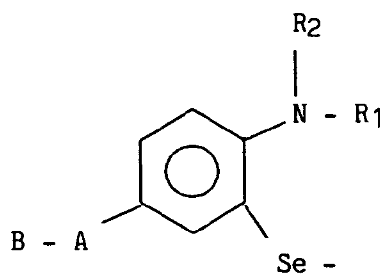

ou bien :

$R_4$ forme avec $R_1$ un groupement -C = O,

– $R_2$ représente un atome d'hydrogène ou un groupement alkyle inférieur éventuellement substitué par un ou plusieurs atomes d'halogène ou par un groupement hydroxy, ou lorsque $R_1$ forme avec $R_4$ un groupement CO, par un groupement $NR_5R_8$, dans laquelle $R_8$ et $R_8$, identiques ou différents représentent chacun indépendamment l'un de l'autre un atome d'hydrogène, un groupement alkyle inférieur, un groupement aryle, un groupement aryle substitué, un groupement arylalkyle inférieur, un groupement arylalkyle inférieur substitué,

ou bien $R_8$ et $R_6$ forment avec l'atome d'azote qui les porte un système hétérocyclique,

– A représente un groupement CO, CHOH ou $CH_2$,

– B représente un atome d'hydrogène, un groupement alkyle inférieur, un groupement cycloalkyle de 3 à 8 atomes de carbone, un groupement alcényle inférieur, un groupement alcynyle inférieur, un groupement aryle ou un groupement aryle substitué, un groupement hétéroaryle ou un groupement hétéroaryle substitué, un groupement arylalkyle inférieur, un groupement hétéroarylalkyle inférieur ou un groupement hétéroarylalkyle inférieur substitué, un groupement styryle ou un groupement styryle substitué,

ou A et B représentent ensemble un atome d'hydrogène à la condition que, lorsque A et B forment ensemble un atome d'hydrogène

. si $R_1$ forme avec $R_4$ un groupement CO alors $R_2$ ne peut représenter ni groupement méthyle, ni un

atome d'hydrogène,

. si $R_1$ ne forme pas avec $R_4$ un groupement CO alors $R_1$ et $R_2$ ne peuvent simultanément représenter un atome d'hydrogène

étant entendu que :

– le terme hétérocyclique que l'on trouve dans les définitions de $R_1$ et $R_2$ représente un système mono ou bicyclique, chaque cycle comprenant de cinq à six sommets et incluant dans son squelette carboné un ou éventuellement plusieurs hétéroatomes identiques ou différents choisis parmi azote, oxygène ou soufre et éventuellement substitué par un groupement alkyle inférieur, par un groupement aryle, par un groupement aryle substitué par un groupement arylalkyle, par un groupement arylalkyle substitué ; par un groupement hétéroaryle, par un groupement hétéroaryle substitué ; par un ou plusieurs atomes d'halogène, par un groupement alkoxy inférieur,

– le terme substitué affectant les groupements aryle, arylalkyle inférieur, hétéroaryle, styryle dans les définitions de $R_1$, $R_2$ et de B signifie que ceux-ci sont substitués sur la partie aromatique par un ou plusieurs groupements identiques ou différents choisis parmi alkyle inférieur, alkoxy inférieur, hydroxy, trifluorométhyle, amino, carboxyle ou par un ou plusieurs atomes d'halogène,

– par groupement alkyle inférieur, alkoxy inférieur, alkényle inférieur, alcynyle inférieur on entend des groupements linéaires ou ramifiés comprenant de 1 à 6 atomes de carbone,

– par groupement aryle on entend un groupement choisi parmi phényle ou naphtyle,

– par groupement hétéroaryle, on entend un groupement aromatique mono ou bicyclique, chaque cycle comprenant cinq ou six sommets, l'ensemble des deux cycles incluant dans son squelette carboné de un à trois hétéroatomes choisis parmi azote, oxygène ou soufre,

leurs isomères, épimères, diastéréoisomères ainsi que lorsque B comprend un groupement carboxyle ou hydroxyle phénolique ou lorsque $R_1$ représente un atome d'hydrogène leurs sels d'addition à une base pharmaceutiquement acceptable, ainsi que lorsque $R_2$ représente un groupement amine, leurs sels d'addition à un acide pharmaceutiquement acceptable.

Parmi les acides pharmaceutiquement acceptables que l'on peut, le cas échéant, ajouter aux composés de formule (I) qui renferment un groupement aminé, on peut citer à titre non limitatif, les acides chlorhydrique, sulfurique, tartrique, maléique, fumarique, oxalique, méthane-sulfonique, camphorique, etc...

Parmi les bases pharmaceutiquement acceptables que l'on peut le cas échéant ajouter aux composés de formule (I) renfermant un groupement acide carboxylique ou un groupement hydroxyle phénolique pour obtenir un sel, ou lorsque $R_1$ représente un atome d'hydrogène, on peut citer à titre non limitatif, les hydroxydes de sodium, potassium, calcium ou aluminium, les carbonates de métaux alcalins ou alcalino terreux, ou des bases organiques comme la triéthylamine, la benzylamine, la diéthylamine, la tert.butylamine, la dicyclohexylamine, l'arginine etc...

La présente invention a également pour objet le procédé de préparation des composés de formule (I) caractérisé en ce que l'on utilise comme matière première un dérivé de formule (II) :

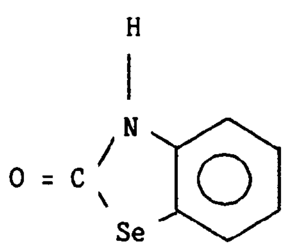

(II)

décrit dans J. Chem. Soc. 1935 (1765) (Hasan et Hunter),

que l'on peut en fonction de la nature de $R_2$ du produit de formule (I) que l'on souhaite obtenir, alkyler à l'azote par un agent d'alkylation pour conduire à un dérivé de formule (II') :

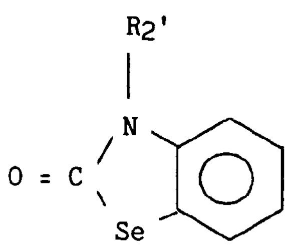

(II')

dans laquelle $R'_2$ représente un groupement alkyle inférieur,
dérivé de formule (II) ou (II'),
que l'on soumet, en fonction de la nature du substituant B du dérivé de formule (I) que l'on souhaite obtenir :
* ou bien à l'action d'un acide de formule (III) :

**B COOH (III)**

dans laquelle B a la même définition que dans la formule (I),
en présence d'acide polyphosphorique,
* ou bien à l'action d'un chlorure d'acide de formule (IV) :

**Cl COB (IV)**

dans laquelle B a la même définition que dans la formule (I),
ou encore à l'action d'un anhydride d'acide de formule (V) :

**$O(COB)_2$ (V)**

dans laquelle B a la même définition que dans la formule (I),
en présence de diméthyl formamide et de chlorure d'aluminium,
pour obtenir un dérivé de formule (I/A) :

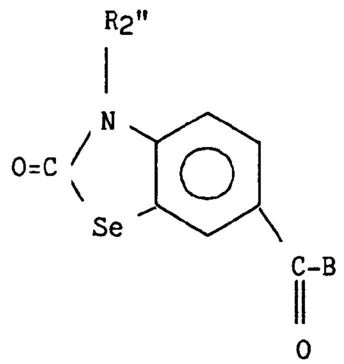

(I/A)

cas particulier des dérivés de formule (I) dans laquelle $R''_2$ représente un atome d'hydrogène ou un groupement alkyle inférieur, B a la même signification que dans la formule (I), A représente un groupement CO, et $R_1$ forme avec $R_4$ un groupement C=O,
que l'on purifie si nécessaire,
que l'on sépare, le cas échéant, en ses isomères et que l'on salifie le cas échéant, si on le désire, par une base pharmaceutiquement acceptable,
dérivé (I/A) que l'on peut, si on le désire, soumettre à l'action d'hydrure mixte de métal alcalin pour conduire à un dérivé de formule (I/B) :

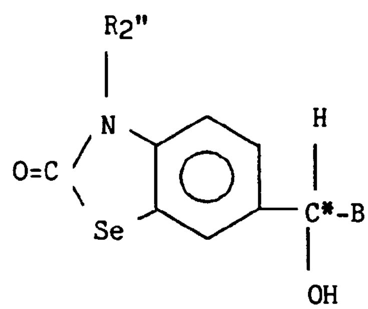

(I/B)

cas particulier des dérivés de formule (I), dans laquelle $R_2''$ a la même signification que précédemment, B a la même signification que dans la formule (I), A représente un groupement CHOH et $R_1$ forme avec $R_4$ un groupement CO,
que l'on purifie si nécessaire,
que l'on sépare, si on le désire en ses isomères et que l'on salifie le cas échéant par une base pharmaceutiquement acceptable,
dérivé de formule (I/A) que l'on peut encore, si on le désire, soumettre à l'action d'un trialkylsilane en milieu acide,
pour conduire à un dérivé de formule (I/C) :

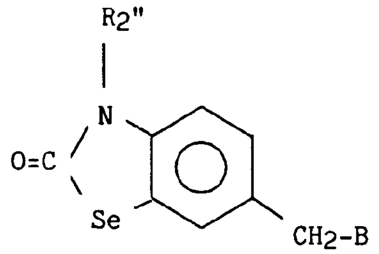

(I/C)

cas particulier des dérivés de formule (I) dans laquelle $R_2''$ et B ont la même signification que précédemment, A représente un groupement $CH_2$ et $R_1$ forme avec $R_4$ un groupement CO, que l'on purifie si nécessaire, que l'on sépare, le cas échéant, en ses isomères et que l'on salifie le cas échéant par une base pharmaceutiquement acceptable,

dérivé de formule (I/A), (I/B) ou (I/C) que l'on peut, lorsque $R_2''$ représente un atome d'hydrogène, traiter :

- ou bien par un agent d'alkylation pour obtenir un dérivé de formule (I/D) :

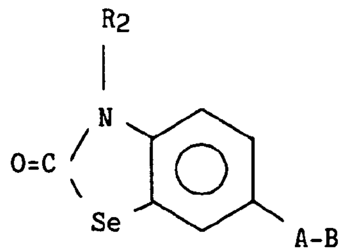

(I/D)

cas particulier des dérivés de formule (I) dans lesquelles A et B ont la même signification que dans la formule (I), $R_2$ représente un groupement alkyle inférieur linéaire ou ramifié,

- ou bien par un agent alcalin puis par un dérivé de formule (VI) :

**$X_1$-M-$X_2$ (VI)**

dans laquelle $X_1$ et $X_2$ différents représentent chacun un atome d'halogène, M représente un groupement alkyle inférieur linéaire ou ramifié pour conduire à un dérivé de formule (I/E) :

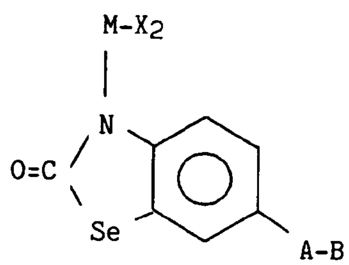

(I/E)

dans laquelle A, B, $X_2$ et M ont la même définition que précédemment, cas particulier des dérivés de formule (I) pour lesquels $R_2$ représente un groupement halogéno alkyle inférieur et $R_1$ forme avec $R_4$ un groupement CO,

que l'on purifie, si nécessaire, et que l'on condense, si on le désire, avec une amine de formule (VIII) :

**$NR_5R_6$ (VIII)**

dans laquelle $R_5$ et $R_5$ ont la même définition que dans la formule (I),

pour obtenir un produit de formule (I/F) :

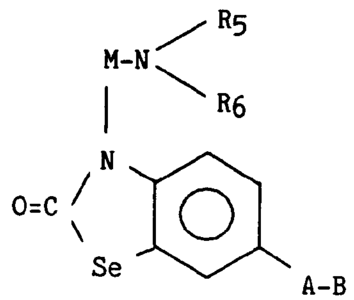

(I/F)

cas particulier des dérivés de formule (I) dans laquelle A et B ont la même signification que dans la formule (I), et $R_2$ représente un groupement alkyle inférieur substitué par un groupement $NR_5R_6$,

- ou bien après action d'un agent alcalin par un dérivé de formule (IX) :

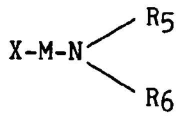

(IX)

dans laquelle X représente un atome d'halogène et M, $R_5$, $R_5$ ont la même signification que précédemment, pour conduire à un dérivé de formule (I/F) que l'on purifie si nécessaire quel que soit le procédé selon lequel il a été obtenu, que l'on sépare, le cas échéant en ses isomères et que l'on salifie, si on le désire par un acide pharmaceutiquement acceptable ou le cas échéant par une base pharmaceutiquement acceptable,

dérivé de formule (I/F) qui, lorsque A représente un groupement CO, peut être soumis, si on le désire à l'action d'un hydrure mixte de métal alcalin pour conduire à un dérivé de formule (I/F1) :

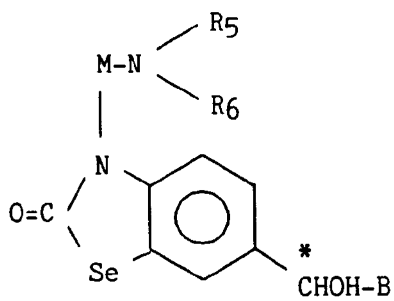

(I/F1)

dans laquelle A représente un groupement CHOH, M, $R_5$, $R_5$ et B ayant la même signification que précédemment, cas particulier des dérivés de formule (I) pour lesquels A représente un groupement CHOH, $R_2$ un groupement alkyle inférieur substitué par un groupement $NR_5R_5$ et $R_1$ forme avec $R_4$ un groupement CO,

que l'on purifie si nécessaire, que l'on sépare en ses isomères, si on le souhaite et que l'on salifie si on le désire par un acide pharmaceutiquement acceptable ou le cas échéant par une base pharmaceutiquement acceptable,

- dérivé de formule (I/A), (I/B), (I/C) ou (I/D) que l'on traite, si on le désire :

. ou bien par un hydroxyde de métal alcalin pour conduire à un dérivé de formule (I/G) :

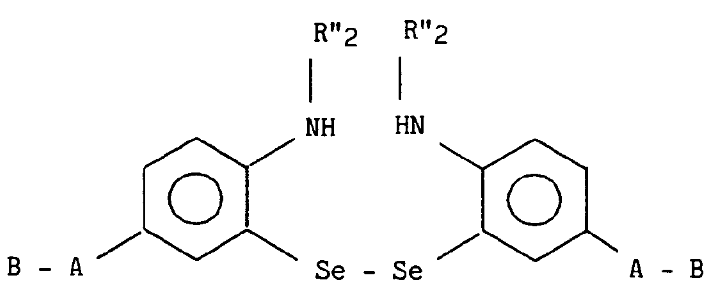

(I/G)

dans laquelle A, B, et $R''_2$ ont la même définition que précédemment, cas particulier des dérivés de formule

(I),

. ou bien par une amine de formule $NR_7R_8$ en présence de formaldéhyde pour conduire à un dérivé de formule (I/H) :

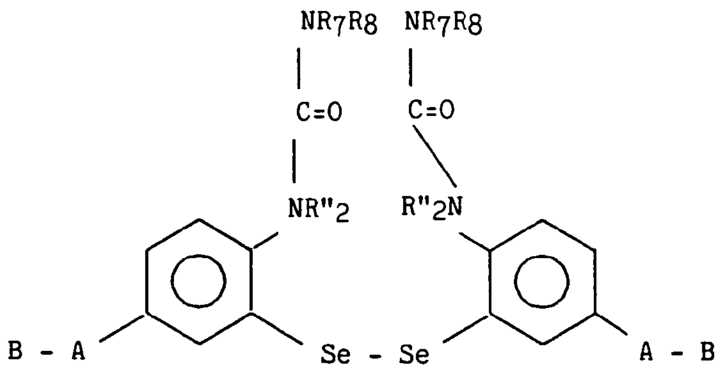

(I/H)

dans laquelle A, B, R"$_2$, $R_7$ et $R_8$ ont la même définition que précédemment, cas particulier des dérivés de formule (I),

dérivé de formule (I/G) ou (I/H) que l'on purifie, si on le désire par une technique classique de chromatographie et/ou cristallisation, que l'on peut le cas échéant séparer en ses isomères, et si on le désire, salifier le cas échéant par une base ou un acide pharmaceutiquement acceptable.

Un cas particulier des dérivés de la présente invention est constitué par les dérivés pour lesquels $R_2$ représente un chaînon méthyle substitué par un groupement $NR_8R_8$ ou un groupement OH et $R_1$ forme avec $R_4$ un groupement C=O. Ces dérivés peuvent être avantageusement obtenus en milieu d'alcool aliphatique inférieur d'un dérivé de formule (II), (I/A), (I/B), (I/C), pour lesquels R"$_2$ représente un atome d'hydrogène et addition d'un excès de formol, chauffage de la solution obtenue à une température comprise entre la température ambiante et la température d'ébullition de la solution, pour conduire, après éventuel refroidissement, repos d'une à deux heures, filtration et éventuelle purification par chromatographie et/ou cristallisation à un dérivé de formule (I/J) :

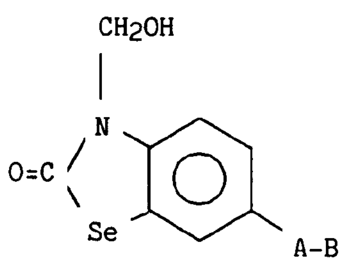

(I/J)

cas particulier des dérivés de formule (I) pour lesquels $R_2$ représente un groupement $CH_2OH$ et $R_1$ forme avec $R_4$ un groupement CO et A et B ont la même définition que dans la formule (I),

que l'on peut traiter par un agent d'halogénation pour conduire à un dérivé de formule (I/E1) :

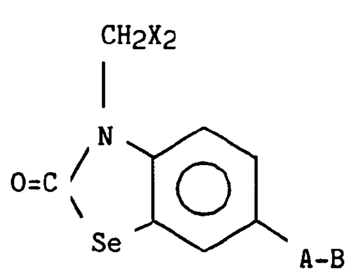

(I/E1)

dans lequel $X_2$ représente un atome d'halogène et A et B ont la même définition que dans la formule (I), cas particulier des dérivés de formule (I/E) pour lequel M représente un groupement $CH_2$, que l'on peut traiter par une amine de formule (VIII) :

**$NR_5R_6$ (VIII)**

dans laquelle $R_5$ et $R_5$ ont la même définition que dans la formule (I), pour conduire à un dérivé de formle (I/F1) :

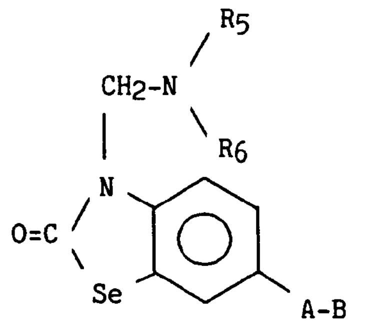

(I/F1)

cas particulier des dérivés de formule (I/F) dans laquelle M représente un groupement $CH_2$ et A, B, $R_5$ et $R_5$ ont la même définition que dans la formule (I),
que l'on purifie, si nécessaire, dont on sépare - le cas échéant les isomères, et que l'on salifie, si on le désire, par un acide ou le cas échéant une base pharmaceutiquement acceptable.

Un autre cas particulier des dérivés de la présente invention concerne les dérivés pour lesquels B représente un groupement styryle ou un groupement styryle substitué, A représente un groupement CO et R1 forme avec R4 un groupement CO. De tels dérivés seront avantageusement obtenus par réaction d'un dérivé de formule (I/I) :

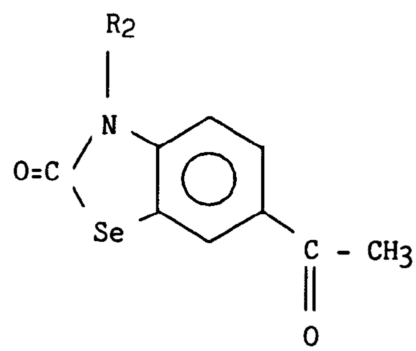

(I/I)

dans lequel $R_2$ a la même définition que dans la formule (I),
avec un aldéhyde benzoïque de formule (X) :

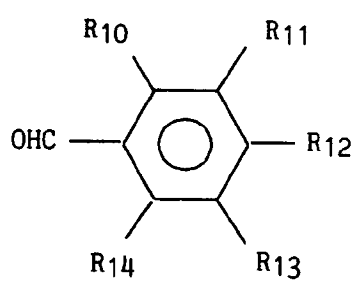

(X)

dans laquelle $R_{10}$, $R_{11}$, $R_{12}$, $R_{13}$, $R_{14}$ identiques ou différents représentent indépendamment l'un de l'autre un atome d'hydrogène, un groupement alkyle inférieur, alkoxy inférieur, hydroxy, trifluorométhyle, carboxy, amino ou atomes d'halogène,
pour conduire à un dérivé de formule (I/S) :

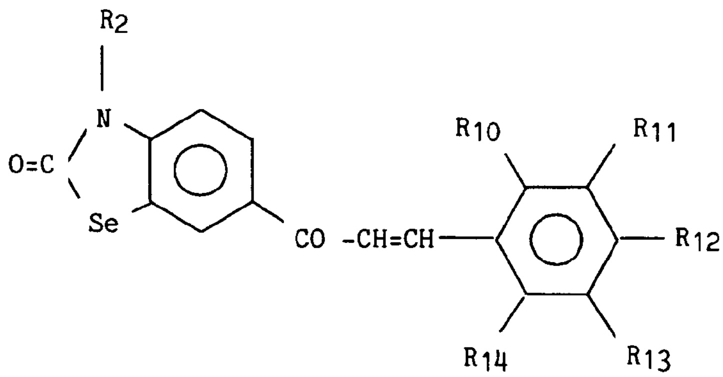

(I/S)

dans laquelle $R_2$, $R_{10}$, $R_{11}$, $R_{12}$, $R_{13}$, $R_{14}$ ont la même définition que précédemment,
que l'on purifie par une technique de chromatographie ou cristallisation dont on sépare les isomères, si on le désire, et que l'on salifie le cas échéant par une base ou un acide pharmaceutiquement acceptable.

L'étude pharmacologique des dérivés de l'invention a en effet montré qu'ils étaient peu toxiques, doués d'activité analgésique antiinflammatoire d'un bon niveau et de remarquables propriétés antioxydantes, antihypoxiques, normolipémiantes, normoglycémiantes, antiagrégantes plaquettaires et immuno stimulantes. Ce spectre d'activité rend les composés de la présente invention intéressants dans un certain nombre d'indications telles que algies rhumatismales, et de façon très générales maladies rhumatismales, névralgies, lombosciatiques, névralgies cervico-brachiales, algies traumatiques telles que entorses, fractures, luxations, douleurs post-traumatiques, douleurs post-opératoires, douleurs neurologiques telles que névralgies faciales, mais encore de la prévention des accidents ischémiques artériels périphériques et cérébro-vasculaires, les troubles hypoxiques ischémiques et oxyprives et donc dans les symptômes du déficit intellectuel, la pathologie du sujet âgé, maladie d'Alzheimer, troubles mnésiques, maladie de Parkinson, les troubles de l'attention, dans la prévention et le traitement des troubles plaquettaires, dans les hyper cholestérolémies, et hypertriglycéridémies, dans le diabète non insulino dépendant ainsi que ses multiples complications telles que cataractes, troubles visuels, névropathies et néphropathies. En tant qu'immunostimulant les produits de l'invention peuvent être utilisés dans le traitement de certains cancers. Leur propriété antioxydante permet également de les utiliser en tant que conservateur notamment d'organes.

La présente invention a également pour objet les compositions pharmaceutiques contenant les produits de formule (I) ou un de leurs sels d'addition à une base pharmaceutiquement acceptable, seuls ou en combinaison avec un ou plusieurs excipients ou véhicules inertes non toxiques, pharmaceutiquement acceptables.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les sachets, les paquets, les gélules, les glossettes, les tablettes, les suppositoires, les crèmes, pommades, gels dermiques, et les solutions de conservation pour organes.

La posologie utile varie selon l'âge et le poids du patient, la voie d'administration, la nature de l'indication thérapeutique ou des traitements éventuellement associés et s'échelonne entre 0,1 centigramme et 4 grammes par 24 heures.

Les exemples suivants illustrent l'invention et ne la limitent en aucune façon.

Les préparations ne font pas partie de l'invention. Toutefois elles constituent des intermédiaires de synthèse intéressants pour l'obtention des produits de l'invention.

**PREPARATION 1 :**

**3-METHYL BENZOSELENAZOLINONE**

Dans un ballon rodé contenant 0,35 grammes (0,015 atome-gramme) de sodium dissous dans 50 $cm^3$ d'alcool absolu, introduire 0,01 mole de benzoselenazolinone. Ajouter goutte à goutte et sous agitation 3,1 $cm^3$ (0,05 mole) d'iodure de méthyle. Après 12 heures, le solvant est évaporé. Le solide obtenu est repris par 30 à 40 $cm^3$ d'eau puis extrait par le chloroforme. La phase chloroformique est séchée sur chlorure de calcium puis on évapore le solvant sous pression réduite. Recristalliser dans le cyclohexane.
Rendement : 80%
Point de fusion : 57-58°C

**PREPARATION 2 :**

**1-(2-CHLORO ETHYL)4-ARYL PIPERAZINES (CHLORHYDRATE)**

A une solution de 0,04 mole d'arylpipérazine dans 40 $cm^3$ de diméthylformamide, ajouter 0,048 mole de carbonate de potassium, puis 0,048 mole de 1-bromo 2-chloro éthane. Agiter à température ambiante pendant 22 heures. Essorer le précipité minéral, et le laver avec du diméthylformamide. Acidifier le filtrat par la quantité suffisante d'alcool absolu saturé d'acide chlorhydrique sec, puis ajouter 300 à 400 $cm^3$ d'éther anhydre. Essorer le précipité de chlorhydrate de 1-(2-chloro éthyl)4-aryl pipérazine.

**EXEMPLE 1 :**

**6-BENZOYL BENZOSELENAZOLINONE**

Introduire dans un ballon de 100 $cm^3$, 1,98 g (0,01 mole) de benzoselenazolinone, 50 à 60 g d'acide polyphosphorique et 1,35 g (0,011 mole) d'acide benzoïque. Chauffer au bain d'huile à une température de 130°C pendant un temps T de 3 heures, sous agitation. Hydrolyser ensuite le mélange réactionnel dans 300 $cm^3$ d'eau froide. Essorer, laver à l'eau, sécher et recristalliser.
Solvant de recristallisation :éthanol à 95
Rendement : 70%
Point de fusion : 204°C
Composition centésimale :

| | | | | | | | |
|---|---|---|---|---|---|---|---|
| Calculée | : | C | 55,64 | H | 3,00 | N | 4,63 |
| Trouvée | : | C | 55,25 | H | 3,00 | N | 4,63 |

**EXEMPLE 2 :**

**3-METHYL 6-BENZOYL BENZOSELENAZOLINONE**

Introduire dans un ballon 50 $cm^3$ d'alcool absolu, 0,35 g de sodium puis 2,8 g (0,1 mole) de 6-benzoyl benzoselenazolinone obtenue dans l'exemple 1. Ajouter goutte à goutte 3,1 $cm^3$ (0,05 mole) d'iodure de méthyle sous agitation. Laisser douze heures à la température du laboratoire. Evaporer à sec. Reprendre le résidu par 30 à 40 $cm^3$ d'eau. Essorer, laver à l'eau, sécher et recristalliser.
Solvant de recristallisation : éthanol à 95
Rendement : 73%
Point de fusion : 156°C
Composition centésimale :

| | | | | | | | |
|---|---|---|---|---|---|---|---|
| Calculée | : | C | 56,97 | H | 3,49 | N | 4,42 |
| Trouvée | : | C | 56,81 | H | 3,50 | N | 4,32 |

**EXEMPLE 3 :**

**6-BUTYRYL BENZOSELENAZOLINONE**

En procédant comme dans l'exemple 1 et en remplaçant l'acide benzoïque par l'acide butyrique, on obtient le produit du titre.
Température de chauffage : 90-95°C
Temps de chauffage : 2H30
Solvant de recristallisation :toluène / acétate d'éthyle 8/2
Rendement : 48%
Point de fusion : 166-167°C
Composition centésimale :

| | | | | |
|---|---|---|---|---|
| Calculée | : | C 49,27 | H 4,13 | N 5,22 |
| Trouvée | : | C 48,86 | H 4,13 | N 5,12 |

**EXEMPLE 4 :**

**3-METHYL 6-BUTYRYL BENZOSENAZOLINONE**

En procédant comme dans l'exemple 2, mais en remplaçant la 6-benzoyl benzosélenazolinone par la 6-butyryl benzoselenazolinone obtenue dans l'exemple 3, on obtient le produit du titre.
Solvant de cristallisation :éthanol - eau (4/1)
Rendement : 70%
Point de fusion : 126-127°C
Composition centésimale :

| | | | | |
|---|---|---|---|---|
| Calculée | : | C 51,08 | H 4,64 | N 4,96 |
| Trouvée | : | C 51,05 | H 4,91 | N 4,96 |

**EXEMPLE 5 :**

**6-ACETYL BENZOSELENAZOLINONE**

Dans une fiole rodée, introduire 12 grammes (0,09 mole) de chlorure d'aluminium. Ajouter goutte à goutte et sous agitation 3,1 $cm^3$ (0,04 mole) de diméthylformamide. Après refroidissement, ajouter deux grammes (0,01 mole) de benzoselenazolinone et le mélange est homogénéisé. Chauffer à 7080°C au bain d'huile sous agitation puis ajouter 0,01 à 0,012 mole de chlorure d'acétyle. Maintenir le chauffage à température de 80-90°C pendant le temps T 4H30 environ. Hydrolyser le mélange sur de la glace pilée. Essorer, sécher et laver par du cyclohexane bouillant, recristalliser dans le toluène.
Rendement : 80%
Point de fusion : 185°C
Composition centésimale :

| | | | | |
|---|---|---|---|---|
| Calculée | : | C 44,72 | H 2,71 | N 5,62 |
| Trouvée | : | C 45,02 | H 2,94 | N 5,83 |

**EXEMPLE 6 :**

**3-METHYL 6-ACETYL BENZOSELENAZOLINONE**

En procédant comme dans l'exemple 2, mais en remplaçant la 6-benzoyl benzoselenazolinone par la 6-acétyl benzoselenazolinone obtenue dans l'exemple 5, on obtient le produit du titre.
Rendement : 87%
Point de fusion : 132-133°C
Composition centésimale :

| | | | | |
|---|---|---|---|---|
| Calculée | : | C 47,26 | H 3,57 | N 5,51 |
| Trouvée | : | C 47,13 | H 3,71 | N 5,34 |

**EXEMPLE 7 :**

**3-METHYL 6-(3',5'-DITERT.BUTYL 4'-HYDROXY) CINNAMOYL BENZOSELENAZOLINONE**

Dans une fiole rodée de 500 ml munie d'une agitation dissoudre 0,01 mole de 6-acétyl 3-méthyl benzoselenazolinone dans 100 ml d'éthanol saturé d'acide chlorhydrique.
Ajouter lentement et sous agitation 0,01 mole de 3,5-ditert.butyl 4-hydroxy benzaldéhyde. Abandonner le milieu

réactionnel à température ambiante pendant deux heures. Verser le milieu réactionnel dans l'eau, essorer le précipité, laver à l'eau jusqu'à neutralité du filtrat et sécher. Recristalliser
Solvant de recristallisation : alcool à 95°
Rendement : 80%
Point de fusion : 203°C
Composition centésimale :

| | | | | |
|---|---|---|---|---|
| Calculée | : | C 63,82 | H 6,21 | N 2,98 |
| Trouvée | : | C 63,48 | H 6,20 | N 3,08 |

**EXEMPLE 8 :**

**6-(3',5'-DITERT.BUTYL 4'-HYDROXY)CINNAMOYL BENZOSELENAZOLINONE**

Procéder comme dans l'exemple 7 en remplaçant la 6-acétyl 3-méthyl benzoselenazolinone par la 6-acétyl benzoselenazolinone.
Solvant de recristallisation : alcool à 95°
Rendement : 75%
Point de fusion : 248-249°C
Composition centésimale :

| | | | | |
|---|---|---|---|---|
| Calculée | : | C 63,15 | H 5,96 | N 3,07 |
| Trouvée | : | C 62,95 | H 5,66 | N 3,24 |

**EXEMPLE 9 :**

**6-(4'-CHLOROBENZOYL) BENZOSELENAZOLINONE**

En procédant comme dans l'exemple 1, mais en remplaçant l'acide benzoïque par l'acide 4-chlorobenzoïque, on obtient le produit du titre.
Temps de chauffage : 4H
Température de chauffage : 130°C
Solvant de recristallisation : éthanol à 95°
Rendement : 70%
Point de fusion : supérieur à 260°C
Composition centésimale :

| | | | | |
|---|---|---|---|---|
| Calculée | : | C 49,95 | H 2,39 | N 4,16 |
| Trouvée | : | C 50,52 | H 2,57 | N 4,17 |

**EXEMPLE 10 :**

**6-NICOTINOYL BENZOSELENAZOLINONE**

Dans une fiole de 250 ml contenant 61,3 g (0,46 mole) de chlorure d'aluminium, introduire goutte à goutte et sous agitation 9,9 ml (0,13 mole) de diméthylformamide, adapter la fiole à un réfrigérant à reflux et porter dans un bain d'huile à une température voisine de 45°C. Introduire 7,9 g (0,04 mole) de benzoselenazolinone et 10,6 g (0,06 mole) de chlorhydrate de chlorure d'acide nicotinique.
Chauffer à la température de 100 °C pendant un temps T (8 heures). Verser le mélange réactionnel dans une quantité suffisante de glace, agiter une heure, essorer le précipité formé, laver à l'eau et sécher. Recristalliser dans l'éthanol.
Rendement : 50%
Point de fusion : 210°C (décomposition)
Composition centésimale :

| | | | | |
|---|---|---|---|---|
| Calculée | : | C 51,50 | H 2,66 | N 9,24 |
| Trouvée | : | C 51,04 | H 2,62 | N 9,07 |

**EXEMPLE 11 :**

**3-METHYL 6-(4'-CHLORO BENZOYL)BENZOSELENAZOLINONE**

Introduire dans un ballon rodé 0,35 grammes (0,015 atome gramme) de sodium dissous dans 50 $cm^3$ d'alcool absolu et 3,40 g (0,01 mole) de 6-(4'-chloro benzoyl) benzoselenazolinone obtenue dans l'exemple 9. Puis goutte à goutte, sous agitation, on ajoute 3,1 $cm^3$ (0,05 mole) d'iodure de méthyle. Après 12 heures, le solvant est évaporé. Reprendre le solide obtenu par 30 à 40 $cm^3$ d'eau et extraire par le chloroforme. Sécher la phase chloroformique sur chlorure de calcium puis évaporer le solvant sous pression réduite. Recristalliser dans l'alcool à 95°.
Rendement : 65%
Point de fusion : 180°C (décomposition)
Composition centésimale :

| | | | | |
|---|---|---|---|---|
| Calculée | : | C 51,38 | H 2,87 | N 3,99 |
| Trouvée | : | C 51,43 | H 2,75 | N 4,06 |

**EXEMPLE 12 :**

**3-METHYL 6-NICOTINOYL BENZOSELENAZOLINONE**

En procédant comme dans l'exemple 5, mais en remplaçant la benzoselenazolinone par la 3-méthyl benzoselenazolinone obtenue dans la préparation 1 et le chlorure d'acétyle par le chlorure de nicotinoyle, on obtient le produit du titre.
Temps de chauffage : 10 à 12 heures
Température : 90°C
Solvant de recristallisation : éthanol à 95°
Rendement : 60%
Point de fusion : 157°C
Composition centésimale :

| | | | | |
|---|---|---|---|---|
| Calculée | : | C 53,01 | H 3,18 | N 8,83 |
| Trouvée | : | C 52,50 | H 3,29 | N 9,03 |

**EXEMPLE 13 :**

**3-METHYL 6-FUROYL BENZOSELENAZOLINONE**

En procédant comme dans l'exemple 10, mais en remplaçant le chlorhydrate de chlorure d'acide nicotinique par le chlorure de 2-furoyle, on obtient le produit du titre.
Caractéristiques spectrales :
1680 $cm^{-1}$ $\nu$ CO SeCON
1650 $cm^{-1}$ $\nu$ CO

**EXEMPLE 14 :**

**ACIDE 4-OXO 4-(BENZOSELENAZOLINON-6-YL)BUTYRIQUE**

Dans une fiole de 250 ml contenant 53,3 g (0,4 mole) de chlorure d'aluminium, introduire goutte à goutte et sous agitation 8,6 ml (0,115 mole) de diméthylformamide. Munir la fiole d'un réfrigérant à reflux et porter dans un bain d'huile à une température voisine de 45°C. Introduire 5,4 g (0,04 mole) de benzoxazolinone et 6 g d'anhydride succinique (0,06 mole). Chauffer à 95°C pendant 5 heures. Verser le mélange réactionnel dans

une quantité suffisante de glace, agiter une heure et essorer le précipité formé.
Reprendre le produit obtenu par une solution aqueuse de bicarbonate de sodium à 10%. Extraire la solution alcaline à plusieurs reprises à l'éther puis acidifier la phase aqueuse par de l'acide chlorhydrique dilué. Essorer le précipité obtenu, laver à l'eau, sécher et recristalliser.
Caractéristiques spectrales :
3300 $cm^{-1}$ ν OH (acide)
1730 $cm^{-1}$ ν CO (acide)
1670 $cm^{-1}$ ν CO (SeCON)
1600 $cm^{-1}$ ν C=C (aromatique)

**EXEMPLE 15 :**

**ACIDE 4-OXO 4-(BENZOSELENAZOLINON-6-YL) BUTENE-2-OÏQUE**

En procédant comme dans l'exemple 14, mais en remplaçant l'anhydride succinique par l'anhydride maléique, on obtient le produit du titre.
Caractéristiques spectrales :
3280 $cm^{-1}$ ν OH (acide)
1670 $cm^{-1}$ ν CO (SeCON)
1720 $cm^{-1}$ ν CO (acide)

**EXEMPLE 16 :**

**ACIDE 2-METHYLENE 4-OXO 4-(BENZOSELENAZOLINO-6-YL) BUTYRIQUE**

En procédant comme dans l'exemple 14, mais en remplaçant l'anhydride succinique par l'anhydride itaconique, on obtient le produit titre.
Caractéristiques spectrales :
Infrarouge :
1670 $cm^{-1}$ ν CO (SeCON)

**EXEMPLE 17 :**

**ACIDE 2-(BENZOSELENAZOLINON-6-YL CARBONYL) BENZOÏQUE**

En procédant comme dans l'exemple 14, mais en remplaçant l'anhydride succinique par l'anhydride phtalique, on obtient le produit du titre.
Caractéristiques spectrales :
Infrarouge :
3460 $cm^{-1}$ ν OH
1670 $cm^{-1}$ ν CO (SeCON)

**EXEMPLE 18 :**

**6-ISONICOTINOYL BENZOSELENAZOLINONE**

En procédant comme dans l'exemple 10, mais en remplaçant le chlorhydrate de chlorure d'acide nicotinique par le chlorhydrate de chlorure d'acide isonicotinique, on obtient le produit du titre.

**EXEMPLE 19 :**

**6-(QUINOL-3-YL-CARBONYL) BENZOSELENAZOLINONE**

En procédant comme dans l'exemple 10, mais en remplaçant le chlorhydrate de chlorure d'acide nicotinique par le chlorhydrate de chlorure d'acide 3-quinoline carboxylique, on obtient le produit du titre.

**EXEMPLE 20 :**

**3-(2-CHLORO ETHYL) BENZOSELENAZOLINONE**

A une solution de 0,015 mole de benzosélénazolinone dans 30 cm3 de diméthylformamide, on ajoute 0,06 mole de carbonate de potassium, puis 0,015 mole de 1-bromo 2-chloro éthane. Le mélange est chauffé à 50°C sous agitation pendant six heures. On essore le précipité minéral, puis on le lave avec du diméthylformamide. Le DMF est évaporé sous pression réduite, et le résidu est repris par de l'eau. On fait alors une extraction par l'éther. La phase organique est séchée sur sulfate de sodium, puis filtrée. La phase éthérée est évaporée à sec sous pression réduite, et le produit est recristallisé dans l'éther de pétrole.
Rendement : 50
Point de fusion : 55°C
Composition centésimale :

| | | | | | | | | |
|---|---|---|---|---|---|---|---|---|
| Calculée | : | C | 41,48 | H | 3,09 | N | 5,37 |
| Trouvée | : | C | 41,61 | H | 2,66 | N | 5,42 |

Caractéristiques Spectrales :
Infrarouge
1650 $cm^{-1}$ : ν CO
Résonance Magnétique Nucléaire ($^1H$) $CDCl_3$
δ : 3,75 ppm, triplet dédoublé 2H, $CH_2$-N
δ : 4,25 ppm, triplet dédoublé 2H, $CH_2$-Cl

**EXEMPLE 21 :**

**3-(3-CHLOROPROPYL) BENZOSELENAZOLINONE**

En procédant comme dans l'exemple 20, mais en remplaçant le 1-bromo 2-chloro éthane par le 1-bromo 3-chloro propane, on obtient le produit du titre.
Rendement : 50%
Point de fusion : 51°C
Composition centésimale :

| | | | | | | | | | | |
|---|---|---|---|---|---|---|---|---|---|---|
| Calculée | : | C | 43,74 | H | 3,67 | N | 5,10 | Cl | 12,91 |
| Trouvée | : | C | 43,69 | H | 3,64 | N | 5,09 | Cl | 12,05 |

Caractéristiques Spectrales :
Infrarouge
1750 $cm^{-1}$ : ν(CH Ar) ; 1640 $cm^{-1}$ ν(CO) ; 2900-2990 $cm^{-1}$ ν($CH_2$)
Résonance Magnétique Nucléaire ($^1H$) $CDCl_3$
δ : 2,25 ppm, 2H, N-$CH_2$
δ : 3,60 ppm, quadruplet, $CH_2$-$CH_2$-$CH_2$
δ : 4,10 ppm, triplet, 2H-$CH_2$-Cl

**EXEMPLE 22 :**

**3-[2-(4-PHENYL PIPERAZIN-1-YL)ETHYL]BENZOSELENAZOLINONE (CHLORHYDRATE)**

**MODE OPERATOIRE 1 :**

Dans une fiole à col rodé, introduire 2 g (0.01) mole de benzoselenazolinone, 2,62 g (0,01 mole) de 1-(2-chloroéthyl) 4-phényl pipérazine (chlorhydrate), 5,52 g (0,04 mole) de carbonate de potassium ainsi que 30 cm3 de diméthylformamide. Chauffer à 50°C sous agitation pendant vingt heures puis essorer. Récupérer le filtrat sous pression réduite et reprendre le résidu par l'eau. Extraire au chloroforme. Récupérer la phase organique, sécher sur sulfate de sodium, filtrer et concentrer, faire barboter un courant d'acide chlorhydrique

gazeux, évaporer à sec. Recristalliser.
Recristallisation : dioxane
Rendement : 50%
Point de fusion : 234°C
Composition centésimale :

| | | | | | |
|---|---|---|---|---|---|
| Calculée | : | C 53,97 | H 5,24 | N 9,94 | Cl 8,38 |
| Trouvée | : | C 53,90 | H 5,36 | N 10,00 | Cl 8,40 |

Caractéristiques Spectrales :
Infrarouge
1650 $cm^{-1}$ : ν(Se CON)
1450 $cm^{-1}$ ν $CH_2$-N
Résonance Magnétique Nucléaire ($^1H$) $CDCl_3$
δ : 2,90 - 4,00 ppm, massif, 10H $CH_2$-N pipérazine
δ : 4,50 ppm, triplet, 2H $CH_2$-N benzoselenazolinone

**MODE OPERATOIRE 2 :**

Ce dérivé peut également être obtenu selon le mode opératoire suivant :

Dans un ballon placé sous argon surmonté d'un réfrigérant, placer 0,01 mole de 3-(2-chloroéthyl)benzoselenazolinone obtenue dans l'exemple 20, un équivalent de 1-phényl pipérazine, 0,04 mole de carbonate de potassium dans 30 cm3 de diméthylformamide. Chauffer à 50 °C sous agitation pendant huit heures, puis essorer. Evaporer le filtrat sous pression réduite et reprendre le résidu par l'éther éthylique. Sécher sur sulfate de sodium, filtrer et concentrer. Abandonner à cristallisation. Essorer, laver à l'éther.

**EXEMPLE 23 :**

**3-{2-[4-(3-TRIFLUOROMETHYL PHENYL)PIPERAZIN-1-YL]ETHYL}BENZOSELENAZOLINONE**

En procédant comme dans l'exemple 22, mode opératoire 2, mais en remplaçant la 1-phényl pipérazine par la 1-(3-trifluorométhyl phényl) pipérazine, on obtient le produit du titre.

**EXEMPLE 24 :**

**3-{2-[4-(2-METHOXY PHENYL)PIPERAZIN-1-YL]ETHYL}BENZOSELENAZOLINONE**

En procédant comme dans l'exemple 22, mode opératoire 2, mais en remplaçant la 1-phényl pipérazine par la 1-(2-méthoxy phényl) pipérazine, on obtient le produit du titre.

**EXEMPLE 25 :**

**3-{2-[4-{4-FLUORO PHENYL)PIPERAZIN-1-YL]ETHYL}BENZOSELENAZOLINONE**

En procédant comme dans l'exemple 22, mode opératoire 2, mais en remplaçant la 1-phényl pipérazine par la 1-(4-fluoro phényl) pipérazine, on obtient le produit du titre.

**EXEMPLE 26 :**

**3-[2-(PYRROLIDIN-1-YL)ETHYL] BENZOSELENAZOLINONE**

En procédant comme dans l'exemple 22, mode opératoire 2, mais en remplaçant la 1-phényl pipérazine par la pyrrolidine, on obtient le produit du titre.

**EXEMPLE 27 :**

**3-(2-MORPHOLINO ETHYL) BENZOSELENAZOLINONE**

En procédant comme dans l'exemple 22, mode opératoire 2, mais en remplaçant la 1-phényl pipérazine par la morpholine, on obtient le produit du titre.
Rendement : 65%
Point de fusion : 80°C
Composition centésimale :

| | | | | | | | | |
|---|---|---|---|---|---|---|---|---|
| Calculée | : | C | 50,17 | H | 5,18 | N | 9,00 |
| Trouvée | : | C | 50,21 | H | 5,24 | N | 9,07 |

Caractéristiques spectrales :
Infrarouge :
2990-2790 $cm^{-1}$ ν CH ($CH_2CH_2$)
1660 $cm^{-1}$ ν CO (NCOSe)
1110 $cm^{-1}$ ν CO ($CH_2$-O-$CH_2$)
Résonance Magnétique Nucléaire :
δ = 7,00 à 7,60 ppm, 4H, massif, aromatiques
δ = 4,10 ppm, 2H, triplet, N-$\underline{CH_2}$-$CH_2$-Morpholine
δ = 3,60 ppm, 4H, massif 2($\underline{CH_2}$-O)
δ = 2,50 ppm, 6H, massif, $\underline{CH_2}$N($\underline{CH_2}$)$_2$ morpholine

**EXEMPLE 28 :**

**3-(2-DIMETHYLAMINO ETHYL) BENZOSELENAZOLINONE (CHLORYDRATE)**

En procédant comme dans l'exemple 22, mode opératoire 1, mais en remplaçant la 1-(2 chloro éthyl)4-phényl pipérazine (chlorhydrate) par le chlorhydrate de 1-chloro 2-diméthyl amino éthane, on obtient le produit du titre.
Solvant de recristallisation : éthanol absolu
Rendement : 55%
Point de fusion : 232°C
Composition centésimale :

| | | | | | | | | | |
|---|---|---|---|---|---|---|---|---|---|
| Calculée | : | C | 43,25 | H | 4,95 | N | 9,17 | Cl | 11,60 |
| Trouvée | : | C | 43,03 | H | 5,00 | N | 9,11 | Cl | 11,48 |

Spectrométrie dans l'infrarouge :
1650 $cm^{-1}$ ν CO SeCON
Spectrométrie de Résonance Magnétique Nucléaire :
δ = 2,30 ppm, singulet, 6H, 2$CH_3$

**EXEMPLES 29 A 34 :**

En procédant comme dans les exemples 22 (mode opératoire 2) 27 à 28, mais en remplaçant la 3-(2-chloroéthyl) benzoselenazolinone par la 3-(3-chloropropyl benzoselenazolinone), on obtient :

**EXEMPLE 29 :**

**3-[3-(4-PHENYL PIPERAZIN-1-YL)PROPYL] BENZOSELENAZOLINONE**

**EXEMPLE 30 :**

**3-{3-[4-(3-TRIFLUOROMETHYLPHENYL)PIPERAZIN-1-YL]PROPYL}BENZOSELENAZOLINONE**

**EXEMPLE 31 :**

**3-{3-[4-(4-FLUOROPHENYL)PIPERAZIN-1-YL]PROPYL}BENZOSELENAZOLINONE**

**EXEMPLE 32** :

**3-[3-(PYRROLIDIN-1-YL)PROPYL]BENZOSELENAZOLINONE**

**EXEMPLE 33 :**

**3-[3-MORPHOLINO PROPYL]BENZOSELENAZOLINONE**

**EXEMPLE 34 :**

**3-[3-DIMETHYLAMINO PROPYL]BENZOSELENAZOLINONE**

**EXEMPLE 35 :**

**3-HYDROXYMETHYL BENZOSELENAZOLINONE**

Dans une fiole à col rodé, introduire 2 grammes (0,01 mole) de benzoselenazolinone, 2 $cm^3$ (0,02 mole) d'une solution aqueuse de formol et 2 $cm^3$ de méthanol. Le mélange est porté deux heures à reflux. Après refroidissement à 40°C, ajouter 20 $cm^3$ d'eau. Laisser refroidir, essorer, laver à l'eau, sécher, recristalliser dans le cyclohexane.
Rendement : 85%
Point de fusion : 105-106°C
Composition centésimale :

| | | | | |
|---|---|---|---|---|
| Calculée | : | C 42,12 | H 3,09 | N 6,14 |
| Trouvée | : | C 42,42 | H 3,33 | N 6,08 |

Caractéristiques spectrales :
Infrarouge :
1660 $cm^{-1}$ $\nu$ CO
Résonance Magnétique Nucléaire $^1H$ : $DMSOD_6$
$\delta$ = 5,30 ppm doublet, 2H, $CH_2OH$

**EXEMPLE 36 :**

**3-BROMOMETHYL BENZOSELENAZOLINONE**

Dans un ballon contenant 0,02 mole de 3-hydroxy méthyl benzoselenazolinone dissoute dans l'acétone, on fait barboter un courant d'acide bromhydrique gazeux sous agitation. Filtrer. Recristalliser dans le cyclohexane.
Rendement : 50%
Point de fusion : 124°C
Caractéristiques spectrales :
Infrarouge :
1660 $cm^{-1}$ $\nu$ CO

Résonance Magnétique Nucléaire $^{1}H$ : ($CDCl_3$)
$\delta$ = 5,70 ppm doublet, 2H, $CH_2Br$

**EXEMPLE 37 :**

**3-CHLOROMETHYL BENZOSELENAZOLINONE**

Dans un ballon contenant 0,01 mole de 3-hydroxyméthyl benzoselenazolinone dissoute dans 100 $cm^3$ de chloroforme, on ajoute goutte à goutte sous agitation 0,55 $cm^3$ de chlorure de thionyle (0,02 mole). Porter à reflux 2 heures puis évaporer le chloroforme sous pression réduite. Recristalliser dans le cyclohexane.
Rendement : 80%
Point de fusion : 97-98°C
Caractéristiques spectrales :
Infrarouge :
1660 $cm^{-1}$ $\nu$ CO
Résonance Magnétique Nucléaire $^{1}H$ :
$\delta$ = 5,70 ppm doublet, 2H, $CH_2Cl$

**EXEMPLE 38 :**

**3-[(4-PHENYL PIPERAZIN-1-YL)METHYL]BENZOSELENAZOLINONE (CHLORHYDRATE)**

Procéder comme dans l'exemple 22, mode opératoire 2 en remplaçant la 3-(2-bromoéthyl)benzoselenazolinone par la 3-bromométhyl benzosélénazolinone. Après reprise du résidu par l'éther éthylique, faire barboter un courant d'acide chlorhydrique gazeux, évaporer à sec et recristalliser.
Caractéristiques spectrales :
Infrarouge
1650 $cm^{-1}$ $\nu$ CO (SeCON)

**EXEMPLE 39 :**

**3-[4-(3-TRIFLUOROMETHYL PHENYL)PIPERAZIN-1-YL METHYL]BENZOSELENAZOLINONE**

En procédant comme dans l'exemple 38, mais en remplaçant la 1-phényl pipérazine par la 1-(3-trifluorométhyl phényl) pipérazine, on obtient le produit du titre.

**EXEMPLE 40 :**

**3-[4-(2-METHOXY PHENYL)PIPERAZIN-1-YL METHYL]BENZOSELENAZOLINONE**

En procédant comme dans l'exemple 38, mais en remplaçant la 1-phényl pipérazine par la 1-(2-méthoxy phényl) pipérazine, on obtient le produit du titre.

**EXEMPLE 41 :**

**3-[4-(4-FLUORO PHENYL)PIPERAZIN-1-YL METHYL]BENZOSELENAZOLINONE**

En procédant comme dans l'exemple 38, mais en remplaçant la 1-phényl pipérazine par la 1-(4-fluoro phényl) pipérazine, on obtient le produit du titre.

**EXEMPLE 42 :**

**3-(PYRROLIDIN-1-YL METHYL) BENZOSELENAZOLINONE**

En procédant comme dans l'exemple 38, mais en remplaçant la 1-phényl pipérazine par la pyrrolidine, on obtient le produit du titre.

**EXEMPLE 43 :**

**3-(MORPHOLINO METHYL) BENZOSELENAZOLINONE**

En procédant comme dans l'exemple 38, mais en remplaçant la 1-phényl pipérazine par la morpholine, on obtient le produit du titre.

**EXEMPLE 44 :**

**3-{DIMETHYLAMINO METHYL) BENZOSELENAZOLINONE**

En procédant comme dans l'exemple 38, mais en remplaçant la 1-phényl pipérazine par la diméthylamine, on obtient le produit du titre.

**EXEMPLE 45 :**

**3-(2-ISOPROPYLAMINO METHYL) BENZOSELENAZOLINONE (CHLORHYDRATE)**

En procédant comme dans l'exemple 22, mais en remplaçant la 1-phényl pipérazine par l'isopropylamine, on obtient le produit du titre. Après reprise du résidu par l'éther éthylique, faire barboter un courant d'acide chlorhydrique gazeux, évaporer à sec et recristalliser.

**EXEMPLE 46 :**

**3-(2-NAPHTYLAMINO METHYL) BENZOSELENAZOLINONE**

En procédant comme dans l'exemple 22 (mode opératoire 2), mais en remplaçant la 1-phényl pipérazine par la naphtylamine, on obtient le produit du titre.

**EXEMPLE 47 :**

**3-(2-BENZYLAMINO ETHYL) BENZOSELENAZOLINONE (CHLORHYDRATE)**

En procédant comme dans l'exemple 22 (mode opératoire 2), mais en remplaçant la 1-phényl pipérazine par la benzylamine, on obtient le produit du titre. Après reprise du résidu par l'éther éthylique, faire barboter un courant d'acide chlorhydrique gazeux, évaporer à sec et recristalliser.

**EXEMPLE 48 :**

**3-[2-(4-CHLORO PHENYL)AMINO ETHYL] BENZOSELENAZOLINONE (CHLORHYDRATE)**

En procédant comme dans l'exemple 22 (mode opératoire 2), mais en remplaçant la 1-phényl pipérazine par la 4-chloro phénylamine, on obtient le produit du titre. Après reprise du résidu par l'éther éthylique, faire barboter un courant d'acide chlorhydrique gazeux, évaporer à sec et recristalliser.

**EXEMPLE 49 :**

**3-[2-(8-AMINO NAPHT-1-YL)AMINO ETHYL] BENZOSELENAZOLINONE**

En procédant comme dans l'exemple 22 (mode opératoire 2), mais en remplaçant la 1-phényl pipérazine par le 1,8-diaminonaphtalène, on obtient le produit du titre. Après reprise du résidu par l'éther éthylique, faire barboter un courant d'acide chlorhydrique gazeux, évaporer à sec et recristalliser.

**EXEMPLE 50 :**

**3-[2-(3-AMINO NAPHT-2-YL)AMINO ETHYL] BENZOSELENAZOLINONE**

En procédant comme dans l'exemple 22 (mode opératoire 2), mais en remplaçant la 1-phényl pipérazine

par le 2,3-diaminonaphtalène, on obtient le produit du titre. Après reprise du résidu par l'éther éthylique, faire barboter un courant d'acide chlorhydrique gazeux, évaporer à sec et recristalliser.

**EXEMPLE 51**

**3-METHYL 6-[(PHENYL)HYDROXYMETHYL] BENZOSELENAZOLINONE**

Dans une fiole placer 0,01 mole de 3-méthyl 6-benzoyl benzoselenazolinone obtenue dans l'exemple 2 dans 50 cm3 de méthanol. Chauffer légèrement et ajouter progressivement 0,1 mole de borohydrure de sodium. Laisser agiter pendant 12 heures. Evaporer le milieu réactionnel au bain-marie sous vide. Laver le résidu à l'eau. Sécher. Recristalliser.

**EXEMPLE 52 :**

**3-METHYL 6-(1-HYDROXY BUTYL) BENZOSELENAZOLINONE**

En procédant comme dans l'exemple 51, mais en remplaçant la 3-méthyl 6-benzoyl benzoselenazolinone par la 3-méthyl 6-butyryl benzoselenazolinone obtenue dans l'exemple 4, on obtient le produit du titre.

**EXEMPLE 53 :**

**3-METHYL 6-(1-HYDROXY ETHYL) BENZOSELENAZOLINONE**

En procédant comme dans l'exemple 51, mais en remplaçant la 3-méthyl 6-benzoyl benzoselenazolinone par la 3-méthyl 6-acétyl benzoselenazolinone obtenue dans l'exemple 6, on obtient le produit du titre.

**EXEMPLE 54 :**

**6-(CYCLOHEXYL CARBONYL) BENZOSELENAZOLINONE**

En procédant comme dans l'exemple 1, mais en remplaçant l'acide benzoïque par l'acide cyclohexane carboxylique, on obtient le produit du titre.

**EXEMPLE 55 :**

**6-(6-METHYL NICOTINOYL) BENZOSELENAZOLINONE**

En procédant comme dans l'exemple 10, mais en remplaçant le chlorure d'acide nicotinique par le chlorure d'acide 6-méthyl nicotinique, on obtient le produit du titre.

**EXEMPLE 56 :**

**6-(PHENYLACETYL) BENZOSELENAZOLINONE**

En procédant comme dans l'exemple 1, mais en remplaçant l'acide benzoïque par l'acide phényl acétique, on obtient le produit du titre.

**EXEMPLE 57 :**

**3-(2-CHLORO ETHYL) 6-BENZOYL BENZOSELENAZOLINONE**

En procédant comme dans l'exemple 20, mais en remplaçant la benzoselenazolinone par la 6-benzoyl benzoselenazolinone, on obtient le produit du titre.

**EXEMPLES 58 A 63 :**

En procédant comme dans les exemples 22 (mode opératoire 2), 23 à 28, mais en remplaçant la 3-(2-chloroéthyl) benzoselenazolinone par la 6-benzoyl 3-(2- chloroéthyl) benzoselenazolinone, on obtient :

**EXEMPLE 58 :**

**3-[2-(4-PHENYL PIPERAZIN-1-YL)ETHYL] 6-BENZOYL BENZOSELENAZOLINONE**

**EXEMPLE 59 :**

**3-{2-[4-(3-TRIFLUOROMETHYL PHENYL ) PIPERAZIN-1-YL]ETHYL} 6-BENZOYL BENZOSELENAZOLINONE**

**EXEMPLE 60 :**

**3-{2-[4-(4-FLUOROPHENYL) PIPERAZIN-1-YL]ETHYL} 6-BENZOYL BENZOSELENAZOLINONE**

**EXEMPLE 61 :**

**3-[2-(PYRROLIDIN-1-YL)ETHYL] 6-BENZOYL BENZOSELENAZOLINONE**

**EXEMPLE 62 :**

**3-(2-MORPHOLINO ETHYL) 6-BENZOYL BENZOSELENAZOLINONE**

**EXEMPLE 63 :**

**3-(2-DIMETHYLAMINO ETHYL) 6-BENZOYL BENZOSELENAZOLINONE**

**EXEMPLE 64 :**

**BIS {2-(3,3-DIMETHYL UREIDO)PHENYL}DISELENIURE**

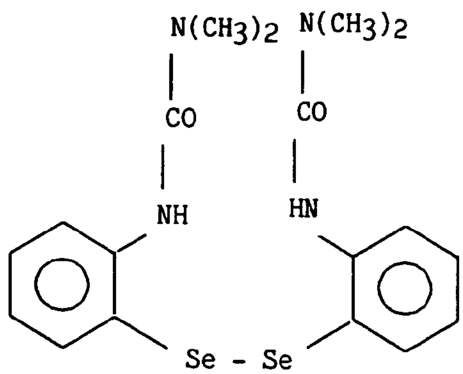

Dans une fiole à col rodé de 50 $cm^3$, on introduit 0,01 mole de benzoselenazolinone ainsi que 18 $cm^3$ d'une solution de diméthylamine à 40% (0,015 mole). Agiter à température ambiante, puis ajouter 0,011 mole de formol. Après deux heures, ajouter 1 $cm^3$ d'alcool à 95°C et porter à reflux 12 heures sous agitation. Après refroidissement, ajouter 1 à 3 $cm^3$ d'alcool à 95°C et maintenir l'agitation un mois. Purifier par flash chromatographie sur colonne de gel de silice : éluant chloroforme 8/acétate d'éthyle 2 pour éliminer les impuretés puis, méthanol pour obtenir le produit. Recristalliser dans l'éther éthylique.

Rendement : 50%

Point de fusion : 116°C

Composition centésimale :

| | | | | | | | | |
|---|---|---|---|---|---|---|---|---|
| Calculée | : | C | 44,64 | H | 4,58 | N | 11,57 |
| Trouvée | : | C | 44,60 | H | 4,51 | N | 11,29 |

**EXEMPLE 65 :**

**BIS {(2-AMINO 5-BENZOYL)PHENYL}DISELENIURE**

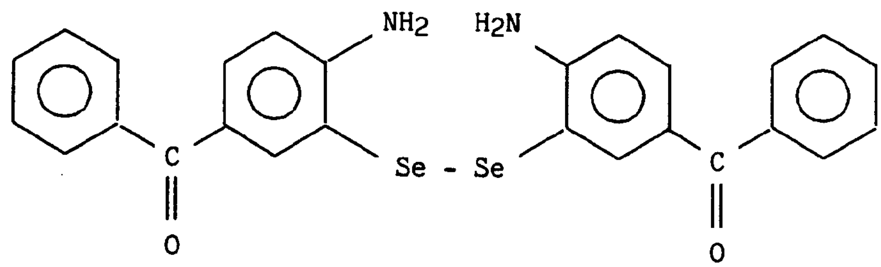

Dans une fiole rodée, on introduit 1,5 g (0,005 mole) de 6-benzoyl benzoselenazolinone ainsi que 20 $cm^3$ de soude à 10%. Chauffer le mélange à reflux sous agitation pendant deux heures. Après refroidissement, on neutralise le milieu réactionnel. Essorer le précipité, laver à l'eau, sécher, recristalliser dans le toluène.
Rendement : 80%
Point de fusion : 190°C
Composition centésimale :

| | | | | |
|---|---|---|---|---|
| Calculée | : | C 56,74 | H 3,66 | N 5,09 |
| Trouvée | : | C 56,57 | H 3,56 | N 5,02 |

**EXEMPLE 66 :**

**BIS {(2-METHYLAMINO 5-BENZOYL) PHENYL}DISELENIURE**

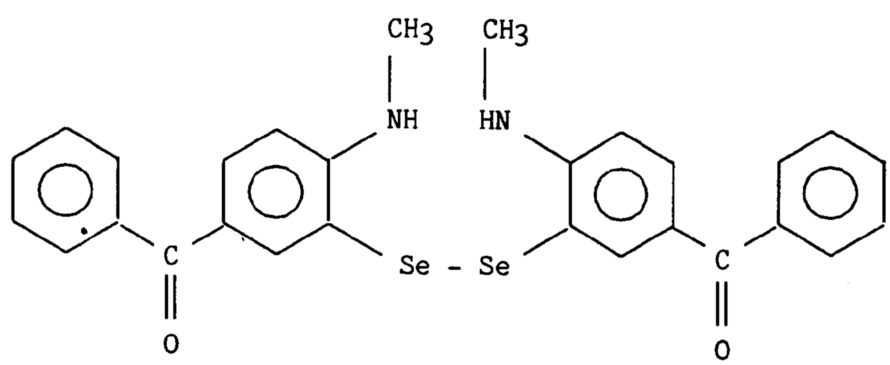

En procédant comme dans l'exemple 65, mais en remplaçant la 6-benzoyl benzoselenazolinone par la 3-méthyl 6-benzoyl benzoselenazolinone, on obtient le produit du titre.
Solvant de recristallisation : Alcool absolu
Rendement : 60%
Point de fusion : 199-200°C
Composition centésimale :

| | | | | |
|---|---|---|---|---|
| Calculée | : | C 58,14 | H 4,18 | N 4,84 |
| Trouvée | : | C 57,97 | H 4,24 | N 4,89 |

Caractéristiques Spectrales :
Infrarouge
1625 $cm^{-1}$ : ν CO (benzoyl)

3310-3320 $cm^{-1}$ : $\nu NH_2$
Résonance Magnétique Nucléaire ($^1H$) $(CD_3)_2SO$
$\delta$ : 2,85 ppm, doublet 6H, $CH_3$

**EXEMPLE 67 :**

**BIS {(2-AMINO 5-ACETYL) PHENYL}DISELENIURE**

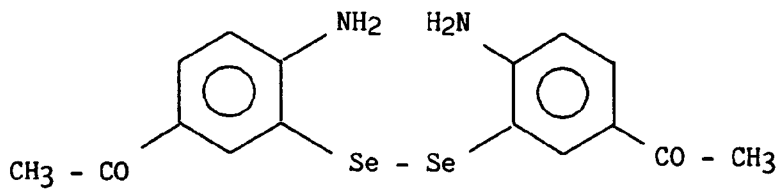

En procédant comme dans l'exemple 65, mais en remplaçant la 6-benzoyl benzoselenazolinone par la 6-acétyl benzoselenazolinone, on obtient le produit du titre.
Solvant de recristallisation : Acétone
Rendement : 40%
Point de fusion : 238°C
Caractéristiques Spectrales :
Infrarouge
1645 $cm^{-1}$ : $\nu$ CO (acétyl)
3450 $cm^{-1}$ : $\nu$ $NH_2$
Résonance Magnétique Nucléaire ($^1H$) $(CD_3)_2SO$
$\delta$ : 2,20 ppm, singulet 6H, $CH_3$

**EXEMPLE 68 :**

**BIS {[2-(3,3-DIMETHYLUREIDO)5-BENZOYL] PHENYL}DISELENIURE**

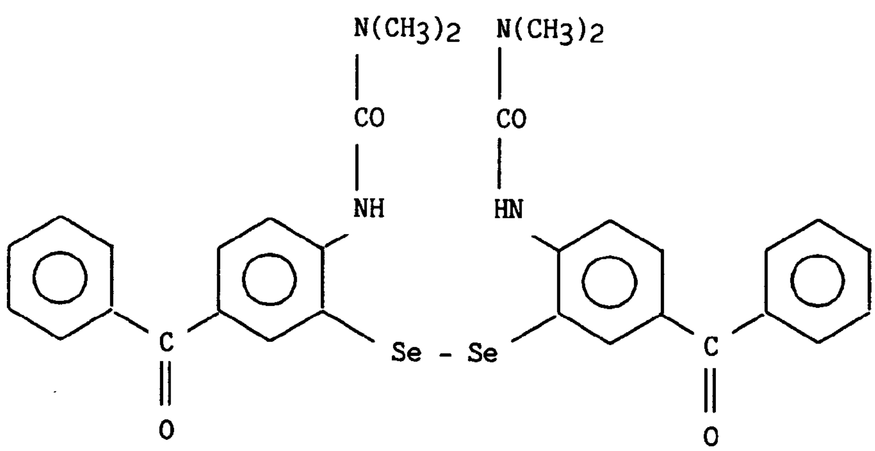

Dans un ballon rodé de 50 $cm^3$, on introduit 3 g (0,01 mole) de 6-benzoyl benzosélénazolinone ainsi que 1,8 $cm^3$ (0,015 mole) d'une solution aqueuse de diméthylamine à 40% et 5 $cm^3$ d'alcool à 95°. L'ensemble est mélangé pendant quelques minutes, puis on ajoute 1 $cm^3$ (0,011 mole) d'une solution aqueuse de fomaldéhyde à 37%. On agite 2 heures à température ambiante, puis on chauffe le mélange à 80°C pendant 15 heures. Après refroidissement, on essore le précipité formé spontanément. Au filtrat on ajoute 1,2 $cm^3$ de diméthylamine, et on porte à reflux sous agitation pendant 9 heures. Après refroidissement, on essore le précipité formé spontanément. Les deux précipités sont réunis, et on purifie le produit à obtenir par chromatographie sur colonne de gel de silice en utilisant le mélange Chloroforme 4/ Acétate d'éthyle 2 comme solvant d'élution.
Rendement : 50%
Point de fusion : 194-195°C

Composition centésimale :

| | | | | |
|---|---|---|---|---|
| Calculée | : | C 55,50 | H 4,37 | N 8,09 |
| Trouvée | : | C 55,37 | H 4,09 | N 7,98 |

Caractéristiques Spectrales :
Infrarouge
1625 $cm^{-1}$ : ν CO (benzoyl)
1680 $cm^{-1}$ : ν CO (carbamoyl)
3250 $cm^{-1}$ : ν CO (NHCO)
Résonance Magnétique Nucléaire ($^1H$) $(CD_3)_2SO$
δ : 2,85 ppm, singulet 12H, $CH_3$

**EXEMPLE 69 :**

**BIS {2-{3,3-DICYCLOHEXYL UREIDO)-PHENYL}DISELENIURE**

En procédant comme dans l'exemple 64, mais en remplaçant la diméthylamine par la dicyclohexylamine, on obtient le produit du titre.

**EXEMPLE 70 :**

**BIS {2-[3-(4-TRIFLUOROMETHYLBENZYL)UREIDO]PHENYL}DISELENIURE**

En procédant comme dans l'exemple 64, mais en remplaçant la diméthylamine par la 4-trifluorométhylbenzylamine, on obtient le produit du titre.

**EXEMPLE 71 :**

**BIS {2-(3-METHYL 3-BENZYL UREIDO]PHENYL}DISELENIURE**

En procédant comme dans l'exemple 64, mais en remplaçant la diméthylamine par la N-méthyl N-benzylamine, on obtient le produit du titre.

**EXEMPLE 72 :**

**BIS {2-[3-(4-CHLOROPHENYL)UREIDO]PHENYL}DISELENIURE**

En procédant comme dans l'exemple 64, mais en remplaçant la diméthylamine par la 4-chloro aniline, on obtient le produit du titre.

**EXEMPLE 73 :**

**BIS {2-(MORPHOLINO CARBONYL AMINO) PHENYL}DISELENIURE**

En procédant comme dans l'exemple 64, mais en remplaçant la diméthylamine par la morpholine, on obtient le produit du titre.

**EXEMPLE 74 :**

**6-ETHYL BENZOSELENAZOLINONE**

Dans une fiole à col rodé de 100 $cm^3$ peser 0,01 mole de 6-acétyl benzoselenazolinone obtenue dans l'exemple 5 et 20 $cm^3$ (0,27 mole) d'acide trifluoroacétique. Par l'intermédiaire d'une ampoule à brome munie d'un robinet en téflon, ajouter sous agitation magnétique, goutte à goutte et en refroidissant au moyen d'un bain d'eau glacée, 5,5 $cm^3$ (0,022 mole) de triéthylsilane. Poursuivre l'agitation du milieu réactionnel pendant 30 heures à température ambiante. Verser, sous agitation le mélange réactionnel dans un litre d'eau glacée.

Essorer le précipité obtenu, laver à l'eau jusqu'à neutralité des eaux de lavage. Sécher. Recristalliser dans le cyclohexane.
Rendement : 80%
Point de fusion : 128°C (décomposition)
Composition centésimale :

| | | | | | | | |
|---|---|---|---|---|---|---|---|
| Calculée | : | C | 47,80 | H | 4,01 | N | 6,19 |
| Trouvée | : | C | 48,20 | H | 4,06 | N | 6,21 |

Spectrométrie dans l'infrarouge :
1660 $cm^{-1}$ ν CO (SeCON)
Spectrométrie de Résonance Magnétique Nucléaire : ($CDCl_3$)
δ : 1,25 ppm, triplet 3H, $CH_3$
δ : 2,5 ppm, quadruplet, 2H, $CH_2$

**EXEMPLE 75 :**

**3-METHYL 6-ETHYL BENZOSELENAZOLINONE**

En procédant comme dans l'exemple 74, mais en remplaçant la 6-acétyl benzoselenazolinone par la 3-méthyl 6-acétyl benzoselenazolinone, on obtient le produit du titre.

**EXEMPLE 76 :**

**6-BENZYL BENZOSELENAZOLINONE**

En procédant comme dans l'exemple 74, mais en remplaçant la 6-acétyl benzoselenazolinone par la 6-benzoyl benzoselenazolinone, on obtient le produit du titre.
Rendement : 95%
Point de fusion : 125-127°C
Composition centésimale :

| | | | | | | | |
|---|---|---|---|---|---|---|---|
| Calculée | : | C | 58,34 | H | 3,85 | N | 4,86 |
| Trouvée | : | C | 58,62 | H | 3,87 | N | 4,80 |

Spectrométrie dans l'infrarouge :
1640 $cm^{-1}$ ν CO (SECON)
Spectrométrie de Résonance Magnétique Nucléaire : ($CDCl_3$)
δ : 4,00 ppm, singulet 2H, $CH_2$
δ : 7,00 à 7,25 ppm, massif, 8H, aromatiques

**EXEMPLE 77 :**

**6-(1-HYDROXY ETHYL) BENZOSELENAZOLINONE**

Dans un ballon rodé refroidi par un bain de glace, on introduit 1,2 g (0,005 mole) d'acétyl-6 benzoselenazolinone ainsi que 7,5 cm3 de soude à 3%. Puis lentement sous agitation, on ajoute 0,38 g (0,001 mole) de borohydrure de sodium. La solution est agitée à température ambiante pendant 15 heures, puis acidifiée par une solution aqueuse d'acide chlorhydrique diluée au 1/4. Le précipité obtenu est essoré, lavé à l'eau, puis recristallisé dans un mélange de cyclohexane (2/3) et d'acétate d'éthyle (1/3).
Rendement : 80%
Point de fusion : 171°C
Composition centésimale :

| | | | | |
|---|---|---|---|---|
| Calculée | : | C 44,64 | H 3,75 | N 5,78 |
| Trouvée | : | C 44,91 | H 3,89 | N 6,03 |

Spectrométrie dans l'infrarouge :
1660 $cm^{-1}$ ν CO
3400 $cm^{-1}$ ν OH
Spectrométrie de Résonance Magnétique Nucléaire : $^1H$, DMSO
δ : 1,25 ppm, doublet 3H, $CH_3$
δ : 4,65 ppm, doublet dédoublé, CHOH

**EXEMPLE 78 :**

**6-[(PHENYL)HYDROXYMETHYL] BENZOSELENAZOLINONE**

**MODE OPERATOIRE**

Dans un ballon rodé refroidi par un bain de glace, on introduit 6 g (0,0198 mole) de benzoyl-6 benzosélénazolinone ainsi que 30 $cm^3$ de soude à 3%. Puis lentement sous agitation, on ajoute 1,14 g (0,030 mole) de borohydrure de sodium. La solution est agitée à température ambiante pendant 6 heures, puis acidifiée par une solution aqueuse d'acide chlorhydrique diluée au 1/4. Le précipité obtenu est essoré, lavé à l'eau, séché, puis recristallisé dans un mélange de cyclohexane (2/3) et d'acétate d'éthyle (1/3).
Rendement : 80%
Point de fusion : 126°C
Composition centésimale :

| | | | | |
|---|---|---|---|---|
| Calculée | : | C 55,28 | H 3,64 | N 4,60 |
| Trouvée | : | C 55,11 | H 3,77 | N 4,55 |

Spectrométrie dans l'infrarouge :
1675 $cm^{-1}$ ν CO
Spectrométrie de Résonance Magnétique Nucléaire : $^1H$, DMSO
δ : 5,60 ppm, singulet $^1H$ CHOH

**EXEMPLE 79 :**

**BIS {2-AMINO 5-(4-CHLOROBENZOYL)PHENYL} DISELENIURE**

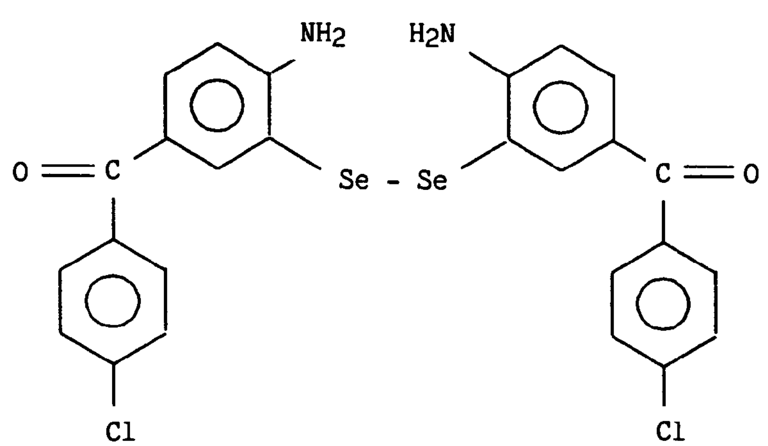

En procédant comme dans l'exemple 65, mais en remplaçant la 6-benzoyl benzosélénazolinone par la 6-(4-chloro benzoyl) benzosélénazolinone, on obtient le produit du titre.
Solvant de recristallisation : isopropanol

Rendement : 60%
Point de fusion : 191°C
Composition centésimale :

| | | | | | |
|---|---|---|---|---|---|
| Calculée | : | C 50,43 | H 2,93 | N 4,52 | Cl 11,45 |
| Trouvée | : | C 50,66 | H 2,98 | N 4,51 | Cl 11,29 |

Caractéristiques spectrales
Infrarouge:
1600 $cm^{-1}$ ν CO parachlorobenzoyl
Spectrométrie de Résonance Magnétique Nucléaire : $^1H$, $(CD_3)_2SO$
δ : 6,57 ppm, singulet 4H pic large $NH_2$
δ : 6,83 ppm, doublet 2H $H_3$ et 3′

**EXEMPLE 80 :**

**BIS {(2-AMINO 5-BENZYL)PHENYL} DISELENIURE**

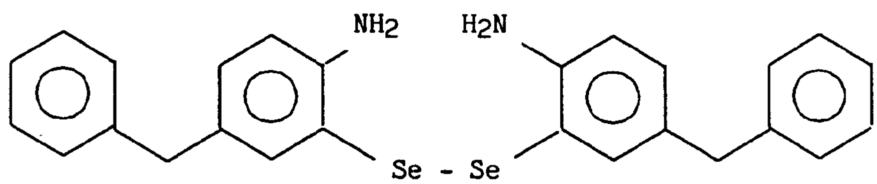

En procédant comme dans l'exemple 65, mais en remplaçant la 6-benzoyl benzosélénazolinone par la 6-benzyl benzosélénazolinone, on obtient le produit du titre.
Solvant de recristallisation : éthanol 95°
Rendement : 70%
Point de fusion : 101°C
Composition centésimale :

| | | | | |
|---|---|---|---|---|
| Calculée | : | C 59,78 | H 4,63 | N 5,36 |
| Trouvée | : | C 59,80 | H 4,56 | N 5,37 |

Caractéristiques spectrales
Infrarouge :
3280 $cm^{-1}$, 3380 $cm^{-1}$ ν NH
Résonance Magnétique Nucléaire : $^1H$, $(CD_3)_2SO$
δ : 3,76 ppm, singulet, 4H $CH_2$

**EXEMPLE 81 :**

**BIS {2-AMINO 5-[(PHENYL)HYDROXY METHYL] PHENYL} DISELENIURE**

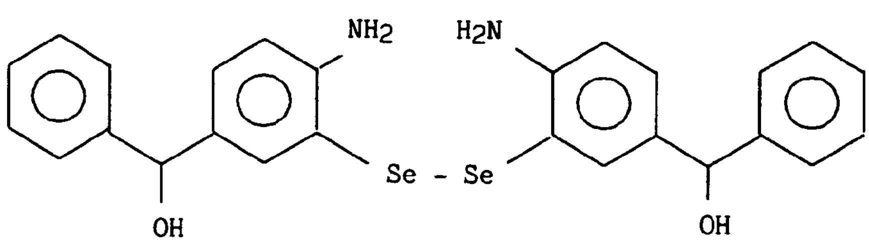

En procédant comme dans l'exemple 65, mais en remplaçant la 6-benzoyl benzosélénazolinone par la 6-(phényl) hydroxy méthyl benzosélénazolinone, on obtient le produit du titre.
Solvant de recristallisation : toluène
Rendement : 50%
Point de fusion : > 270°C
Composition centésimale :

| | | | | |
|---|---|---|---|---|
| Calculée | : | C 56,33 | H 4,36 | N 5,05 |
| Trouvée | : | C 56,47 | H 4,34 | N 4,97 |

Spectrométrie dans l'infrarouge :
1600 $cm^{-1}$ ν CHAr
3340 $cm^{-1}$ ν CH + OH
Spectrométrie de Résonance Magnétique Nucléaire : $^1H$, $(CD_3)_2SO$
δ : 5,50 ppm, doublet 2H CHOH

**EXEMPLE 82 :**

**BIS [(2-METHYL AMINO)PHENYL] DISELENIURE**

En procédant comme dans l'exemple 65, mais en remplaçant la 6-benzoyl benzosélénazolinone par la 3-méthyl benzosélénazolinone, on obtient le produit du titre.
Point de fusion : 88°C

**ETUDE PHARMACOLOGIQUE DES PRODUITS DE L'INVENTION**

**EXEMPLE 83 :**

**TOXICITE AIGUE**

La toxicité aigue a été appréciée après administration orale à des lots de cinq souris (20 ± 2 grammes) de doses croissantes (0,1 - 0,25 - 0,50, 0,75 - 1 g/kg). Les animaux ont été observés à intervalles réguliers au cours de la première journée et quotidiennement pendant les deux semaines suivants le traitement.
Il apparaît que les composés de l'invention sont totalement atoxiques. Aucun décès n'est observé après administration d'une dose de 1 $g.kg^{-1}$. On ne constate pas de trouble après administration de cette dose.

**EXEMPLE 84 :**

**MESURE DE L'ACTIVITE DE LA GLUTATHION PEROXYDASE (GPx)**

La glutathion peroxydase est une enzyme qui catalyse la réduction de péroxydes en alcools. Les produits séléniés ont une activité de type glutathion peroxydase.
La réaction utilise le glutathion réduit (GSH) comme cofacteur et produit du glutathion oxydé (GSSG) (Phase 1). Un excès de glutathion réductase associé à du NADPH sous forme réduite comme cofacteur permet de maintenir la concentration de glutathion réduit à son niveau initial.
Ainsi, l'oxydation de NADPH observée en UV à 340 nm est directement liée à l'activité glutathion péroxydase.
Cette méthode est réputée fiable pour comparer les activités de différents produits (A. WENDEL, Glutathione peroxydase. Methods in Enzymol. 1981, 77, 325-333 et B. FARAJI, H.K. KANGE et J.L. VALENTINE, Methods compared for determining glutathione peroxidase activity in blood. Clin. Chem. 33, 539-549, 1987).

$$ROOH + 2\ GSH \xrightarrow{GPx} ROH + GSSG + H_2O \qquad (I)$$

$$GSSG + NADPH + H^+ \xrightarrow{GSH\ red} 2GSH + NADP+ \qquad (II)$$

$$ROOH + NADPH + H^+ \longrightarrow ROH + H_2O + NADP^+$$

Certains des produits de l'invention ont montré une activité glutathion peroxydase approximativement trois fois plus intense que celle du dérivé faisant l'objet de la demande de brevet européen N° 0 044 971.

**EXEMPLE 85 :**

**MISE EN EVIDENCE D'UNE ACTIVITE ANTILIPOPEROXYDANTE**

Après décapitation les hémisphères cérébraux de rats males Wistar sont homogénéisés. L'homogénat est incubé seul 30 minutes à 37°C pour l'étude de la peroxydation spontanée et en présence d'un système générateur de radicaux libres, Fer-Ascorbate, pour l'étude de la péroxydation induite. L'intensité de la peroxydation lipidique en présence et en absence des dérivés de l'invention est mesurée par détermination des substances réagissant avec l'acide thiobarbiturique (SRTBA) exprimées en mmoles de malondialdéhyde par une méthode dérivée de celle de Yagi (Yagi K. Biochem. Med., 1976, 15,212-216). Il apparaît que les produits de l'invention ont une activité intéressante sur ce test.

**EXEMPLE 86 :**

**ETUDE DE L'ACTIVITE ANTIAGREGANTE PLAQUETTAIRE**

Un plasma riche en plaquettes est préparé à partir de sang humain citraté, provenant de donneurs n'ayant pris aucun médicament pendant les dix jours précédant le prélèvement.
L'agrégation des plaquettes dans ce milieu plasmatique est étudiée par turbidimétrie en employant un agoniste : l'acide arachidonique. Les produits de l'invention sont ajoutés au plasma trois minutes avant l'agoniste.
Les produits de l'invention manifestent une activité antagoniste de l'agrégation plaquettaire significative.

**EXEMPLE 87 :**

**STIMULATION DES REPONSES IMMUNITAIRES**

A des groupes de six souris on a administré des globules rouges de moutons. Ces groupes de souris ont ensuite été traités par voie sous cutanée par les composés de l'invention pendant six jours et un groupe témoin a été traité par un placebo. Les souris sont ensuite laissées au repos pendant quatre semaines puis ont ensuite reçu une injection de rappel de globules rouges de mouton sans recevoir de nouvelles administrations de produit de l'invention. La réponse immunitaire a été évaluée 3 jours après l'injection de rappel. Elle est statistiquement accrue dans le groupe traité par les composés de l'invention.

**EXEMPLE 88 :**

**ETUDE DE L'ACTIVITE HYPOGLYCEMIANTE**

Des souris mâles KK ont été placées dans des cages à l'âge de huit semaines. Elles sont utilisées pour l'expérience lorsque leur poids est supérieur à 40 grammes à l'âge de 4-5 mois.

Le composé de l'invention est placé en suspension dans du sirop de gomme. Chaque composé testé est administré oralement, 18 heures avant le prélèvement sanguin.

Le sang est recueilli par prélèvement au niveau de la veine caudale dans un tube à hématocrite, puis centrifugé. Le plasma est recueilli et le dosage de la glycémie effectué.

Il apparaît que les composés de l'invention diminuent la glycémie de façon significative.

**EXEMPLE 89 :**

**ETUDE DE L'ACTIVITE ANTIINFLAMMATOIRE**

Mesure de l'inhibition de la cyclooxygénase :

L'activité de la cyclooxygénase est mesurée à partir de plaquettes humaines lavées, en présence et absence des dérivés revendiqués, après activation par la thrombine (Cerletti et Coll., J. Clin. Invest., 1986, 78, 323-326).

Le thromboxane B2 produit est dosé par radio immunologie. Il apparaît qu'à une concentration de $10^{-5}$M certains produits de l'invention antagonisent la cyclooxygénase dans une proportion de 90 à 95%.

Mesure de l'inhibition de la lipoxygénase :

L'activité de la lipoxygénase est mesurée à partir de polynucléaires humains lavés, en présence et absence des dérivés revendiqués, après activation par le calcium (A23187). Le leukotriène B4 (LTB4) produit est mesuré par radioimmunoassay (Gresele, P et Coll. Biochem. Biophys. Res. Comm., 1986, 137, 334-342). Il apparaît qu'à une concentration de $10^{-5}$M, certains produits de l'invention antagonisent la lipoxygénase dans une proportion de 90 à 97%.

Il apparaît que les produits de l'invention sont doués d'activité antiinflammatoire de type mixte étant simultanément inhibiteurs de lipoxygénase et cyclooxygénase.

Inhibition de l'oedème à la carraghénine :

Le potentiel antiinflammatoire des dérivés de l'invention a également été recherché sur un modèle d'inflamation anguë provoquée par injection sous-cutanée d'une solution de carraghénine au niveau de la patte postérieure de rat, selon une technique inspirée de la méthode de WINTER C.A ; E.A. RISLEY, G.N. NUSS - (Proc. Soc. Exp. Med. 111, 554, 1962). Les rats (100-120 g), randomisés en lots de 8, ont été traités par les produits de l'invention (les témoins reçoivent l'excipient) 1 heure avant l'injection locale d'une solution, à 0,5% de carragénine (type IV, Sigma ; 0,1 ml par rat). L'oedème est déterminé 3 heures après l'injection, par mesure pléthysmométrique (pléthysmomètre à eau UGO BASILE) du volume de chacune des pattes postérieures (oedème = volume de la patte enflammée dimunué du volume de la patte non enflammée).

Il apparaît que les produits de l'invention à une dose de 3 $mg.kg^{-1}$ diminuent significativement le volume de l'oedème.

**EXEMPLE 90 :**

**ETUDE DES PROPRIETES ANTIOXYDANTES**

La propriété du chlorhydrate de 2,2'-azao bis (2-amidino propane) (agent d'oxydation) (AAPH), qui consiste à générer des radicaux libres à taux constant, est utilisée pour induire la lyse d'hématies humaines.

L'inhibition de l'hémolyse pour les composés étudiés est calculée par comparaison au pourcentage d'hémolyse obtenu avec le témoin AAPH par dosage de l'hémoglobine libérée par mesure de la densité optique à 403nm. Les produits de l'invention manifestent une intéressante activité sur ce test.

**EXEMPLE 91 :**

**ETUDE DE L'ACTIVITE ANALGESIQUE**

L'activité sur la douleur a été recherchée chez la souris (23-25 g) selon un protocole dérivé de la technique décrite par SIEGMUND (SIEGMUND E.A., R.A. CADMUS & GOLU, J. Pharm. Exp. Ther. 119, 1874, 1954). Les souris, réparties par randomisation en lots de 12 animaux, ont reçu le traitement par voie orale (excipient pour les témoins) 1 heure avant l'injection intra-péritonéale d'une solution hydroalcoolique de phényl-p-benzoquinone (Sigma) à 0,02%. Les étirements sont dénombrés entre la 5ème et 10ème minute après l'injection.

Le pourcentage d'activité obtenu a été évalué pour chaque dose (% de diminution du nombre d'étirements chez les traités par rapport aux témoins). Une $ED_{50}$, dose entraînant une activité de 50%, a été déterminée pour chaque produit.

Il est apparu que certains composés de l'invention possèdent une activité analgésique très intéressante. Ainsi, certains composés de l'invention ont une $ED_{50}$ voisine de 10 $mg.kg^{-1}$.

**EXEMPLE 92 :**

**ACTIVITE SUR LE BILAN LIPIDIQUE**

L'étude de l'effet des produits de l'invention sur le bilan lipidique est réalisée après administration répétée des composés de l'invention par voie orale pendant 7 jours.

L'essai est réalisé sur des groupes de 12 souris Iffa Credo pesant 25±1 g le jour du début du traitement. Un groupe reçoit un placebo. L'autre groupe de souris reçoit le fénofibrate à une dose de 300 $mg.kg^{-1}$/jour. Les autres groupes de souris reçoivent les produits de l'invention à une dose de 30 $mg.kg^{-1}$/jour. Le dosage des triglycérides, cholestérol total, libre et estérifié fait apparaître une diminution significative de ces paramètres supérieure à celle obtenue avec le fénofibrate (à dose 10 fois supérieure).

**EXEMPLE 93 :**

**ETUDE DE L'ACTIVITE ANTIHYPOXIQUE DES DERIVES DE L'INVENTION**

Des rats mâles de poids compris entre 300 et 350 g sont anesthésiés au pentobarbital sodique (60 $mg.kg^{-1}$ i.p.). Les animaux sont héparinés et après ouverture abdominale et section du diaphragme, le coeur est prélevé. Il est alors fixé par l'aorte à la canule de perfusion et perfusé par voie atriale gauche selon le protocole de Rochette et Coll. (American Heart Journal, 107, 1132-1141).

Après 15 minutes de perfusion, la substance à étudier est ajoutée au liquide de perfusion (Krebs) ; elle est maintenue dans ce liquide jusqu'à la fin de l'expérience. Une ligature de l'artère coronaire gauche est réalisée 15 minutes après l'adjonction de la substance. Elle est maintenue durant 10 minutes et la reperfusion faisant suite à la levée de la ligature est étudiée durant 10 minutes.

En présence des produits de l'invention est notée une amélioration significative des paramètres suivants :
- débit coronaire
- débit aortique
- débit cardiaque
- fréquence cardiaque.

**EXEMPLE 94 :**

**CONDITIONS PHARMACEUTIQUES**

**COMPRIMES**

Comprimés dosés à 10 mg de Bis [(2-méthylamino 5-benzoyl)phényldiséléniure

Formule de préparation pour 1000 comprimés :

| | |
|---|---|
| Bis [(2-méthylamino 5- benzoyl)phényl]diséléniure | 10 g |
| Amidon de blé | 15 g |
| Amidon de maïs | 15 g |
| Lactose | 65 g |
| Stéarate de magnésium | 2 g |
| Silice | 1 g |
| Hydroxypropylcellulose | 2 g |

**Revendications**

1. Dérivés de formule générale (I) :

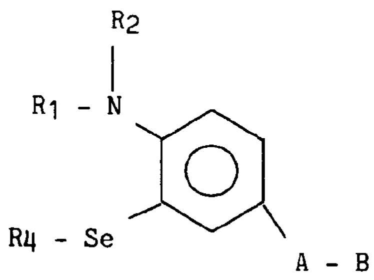

(I)

dans laquelle :

– $R_1$ représente soit un atome d'hydrogène soit un groupement CO-$NR_7R_8$ avec $R_7$, $R_8$ identiques ou différents, représentant un atome d'hydrogène, un groupement alkyle inférieur ou cycloalkyle de 3 à 8 atomes de carbone, ou aryle, ou aryle substitué, ou arylalkyle inférieur, ou arylalkyle inférieur substitué ou forment avec l'azote qui les porte un système hétérocyclique,

– $R_4$ représente un groupement :

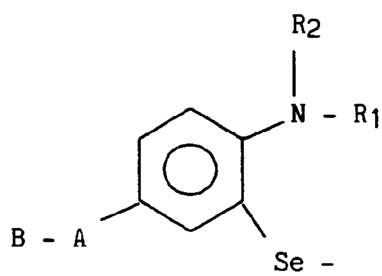

ou bien :

$R_4$ forme avec $R_1$ un groupement -C = O,

– $R_2$ représente un atome d'hydrogène ou un groupement alkyle inférieur éventuellement substitué par un ou plusieurs atomes d'halogène ou par un groupement hydroxy, ou lorsque $R_1$ forme avec $R_4$ un groupement CO, par un groupement $NR_8R_8$, dans laquelle $R_8$ et $R_6$, identiques ou différents représentent chacun indépendamment l'un de l'autre un atome d'hydrogène, un groupement alkyle inférieur, un groupement aryle, un groupement aryle substitué, un groupement arylalkyle inférieur, un groupement arylalkyle inférieur substitué,

ou bien $R_8$ et $R_8$ forment avec l'atome d'azote qui les porte un système hétérocyclique,

– A représente un groupement CO, CHOH ou $CH_2$,

– B représente un atome d'hydrogène, un groupement alkyle inférieur, un groupement cycloalkyle de 3 à 8 atomes de carbone, un groupement alcényle inférieur, un groupement alcynyle inférieur, un groupement aryle ou un groupement aryle substitué, un groupement hétéroaryle ou un groupement hétéroaryle substitué, un groupement arylalkyle inférieur, un groupement hétéroarylalkyle inférieur ou un groupement hétéroarylalkyle inférieur substitué, un groupement styryle, ou un groupement styryle substitué,

– ou A et B représentent ensemble un atome d'hydrogène à la condition que lorsque A et B forment ensemble un atome d'hydrogène :

. si $R_1$ forme avec $R_4$ un groupement CO alors $R_2$ ne peut représenter un groupement méthyle, ou un atome d'hydrogène,

. si $R_1$ ne forme pas avec $R_4$ un groupement CO alors $R_1$ et $R_2$ ne peuvent simultanément représenter un atome d'hydrogène,

étant entendu que :

– le terme hétérocyclique que l'on trouve dans les définitions de $R_1$ et $R_2$ représente un système mono ou bicyclique, chaque cycle comprenant de cinq à six sommets et incluant dans son squelette carboné un ou éventuellement plusieurs hétéroatomes identiques ou différents choisis parmi azote, oxygène ou soufre et éventuellement substitué par un groupement alkyle inférieur, par un groupement aryle, par un groupement aryle substitué par un groupement arylalkyle, par un groupement arylalkyle substitué ; par un groupement hétéroaryle par un groupement hétéroaryle substitué ; par un ou plusieurs atomes d'halogène, par un groupement alkoxy inférieur,

– le terme substitué affectant les groupements aryle, arylalkyle inférieur, hétéroaryle, styryle dans les

définitions de $R_1$, $R_2$ et de B signifie que ceux-ci sont substitués sur la partie aromatique par un ou plusieurs groupements identiques ou différents choisis parmi alkyle inférieur, alkoxy inférieur, hydroxy, trifluorométhyle, amino, carboxyle ou par un ou plusieurs atomes d'halogène,

– par groupement alkyle inférieur, alkoxy inférieur, alkényle inférieur, alcynyle inférieur on entend des groupements linéaires ou ramifiés comprenant de 1 à 6 atomes de carbone,

– par groupement aryle on entend un groupement choisi parmi phényle ou naphtyle,

– par groupement hétéroaryle, on entend un groupement aromatique mono ou bicyclique, chaque cycle comprenant cinq ou six sommets, l'ensemble des deux cycles incluant dans son squelette carboné de un à trois hétéroatomes choisis parmi azote, oxygène ou soufre,

leurs isomères, épimères, diastéréoisomères ainsi que lorsque B comprend un groupement carboxyle ou hydroxyle phénolique ou lorsque $R_1$ représente un atome d'hydrogène leurs sels d'addition à une base pharmaceutiquement acceptable, ainsi que lorsque $R_2$ représente un groupement amine, leurs sels d'addition à un acide pharmaceutiquement acceptable.

2. Composés selon la revendication 1 pour lesquels $R_1$ forme avec $R_4$ un groupement CO, de formule (I/D1) :

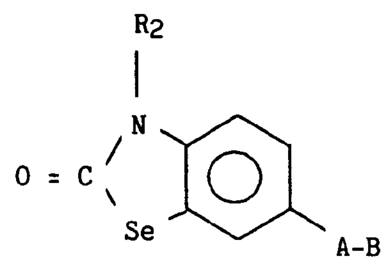

(I/D1)

pour lesquelles $R_2$, A et B ont la même définition que dans la formule (I), leurs isomères ainsi que le cas échéant, leurs sels d'addition à un acide ou une base pharmaceutiquement acceptables.

3. Composés selon revendication 1 de formule (I/G1) :

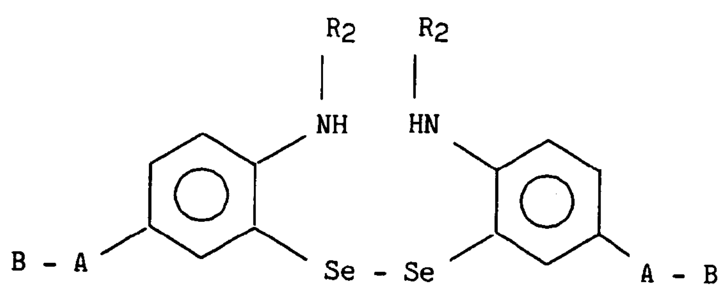

(I/G1)

dans laquelle A, B et R2 ont la même définition que dans la formule (I), leurs isomères ainsi que le cas échéant, leurs sels d'addition à un acide ou une base pharmaceutiquement acceptables.

4. Composés selon la revendication 1 pour lesquels A-B représentent un atome d'hydrogène, leurs isomères ainsi que le cas échéant, leurs sels d'addition à un acide ou une base pharmaceutiquement acceptable.

5. Composés selon la revendication 1 de formule générale (I/F) :

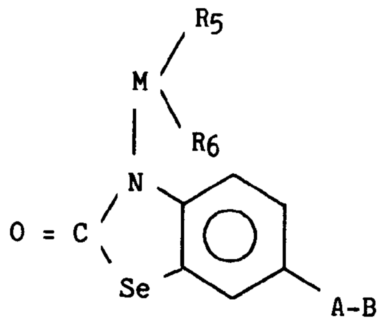

(I/F)

pour lesquels M représente un chaînon alkyle inférieur et A, B, $R_5$ et $R_5$ ont la même définition que dans la formule (I), leurs isomères ainsi que leurs sels d'addition à un acide ou une base pharmaceutiquement acceptables.

**6.** Composés selon la revendication 1, de formule (I/S) :

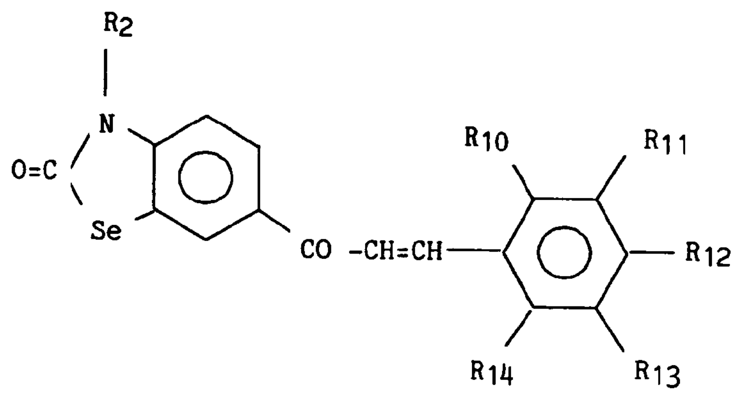

(I/S)

dans laquelle $R_2$ a la même signification que dans la revendication 1 et $R_{10}$, $R_{11}$, $R_{12}$, $R_{13}$ et $R_{14}$ identiques ou différents représentent indépendamment l'un de l'autre un atome d'hydrogène, un groupement alkyle inférieur, alkoxy inférieur, hydroxy, trifluorométhyle, amino ou atome d'halogène, leurs isomères ainsi que le cas échéant, leurs sels d'addition à un acide ou une base pharmaceutiquement acceptable.

**7.** Composés selon la revendication 1 pour lesquels A représente un groupement CO et B représente un groupement phényle, leurs isomères ainsi que le cas échéant, leurs sels d'addition à une base pharmaceutiquement acceptable.

**8.** Composé selon la revendication 1 qui est la 6-(3',5'-ditert.butyl 4'-hydroxy)cinnamoyl benzosélénazolinone ses isomères, ainsi que ses sels d'addition à une base pharmaceutiquement acceptable.

**9.** Composé selon la revendication 1 qui est la 3-méthyl 6-(3',5'-ditertbutyl 4'-hydroxy)cinnamoyl benzoselenazolinone, ses isomères, ainsi que ses sels d'addition à une base pharmaceutiquement acceptable.

**10.** Composé selon la revendication 1 qui est la 6-benzoyl benzosélénazolinone ainsi que ses sels d'addition à une base pharmaceutiquement acceptable.

**11.** Composé selon la revendication 1 qui est le bis [(2-amino 5-benzoyl) phényl] diséléniure ainsi que ses sels d'addition à un acide pharmaceutiquement acceptable.

**12.** Composé selon la revendication 1 qui est le bis [(2-méthyl amino 5-benzoyl) phényl] diséléniure ainsi que ses sels d'addition à un acide pharmaceutiquement acceptable.

**13.** Composé selon la revendication 1 qui est le bis [2-(3,3-diméthyl uréido) phényl] diséléniure.

14. Composé selon la revendication 1 qui est le bis [2-amino 5-(phényl hydroxy méthyl)phényl] diséléniure ainsi que ses sels d'addition à une base pharmaceutiquement acceptable.

15. Composé selon la revendication 1 qui est le bis [2-(3,3-diméthyl uréido)5-benzoyl phényl] diséléniure.

16. Composé selon la revendication 1 qui est le bis [2-amino 5-acétyl phényl] diséléniure ainsi que ses sels d'addition à un acide pharmaceutiquement acceptable.

17. Composé selon la revendication 1 qui est le bis [(2-méthyl amino) phényl]diséléniure ainsi que ses sels d'addition à un acide pharmaceutiquement acceptable.

18. Composé selon la revendication 1 qui est la 6-[(phényl) hydroxy méthyl] benzosélénazolinone ainsi que ses sels d'addition à une base pharmaceutiquement acceptable.

19. Procédé de préparation des composés de formule (I) selon la revendication 1, caractérisé en ce que l'on utilise comme matière première un dérivé de formule (II) :

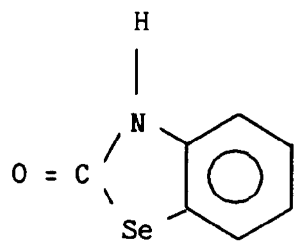

(II)

que l'on peut en fonction de la nature de $R_2$ du produit de formule (I) que l'on souhaite obtenir, alkyler à l'azote par un agent d'alkylation pour conduire à un dérivé de formule (II') :

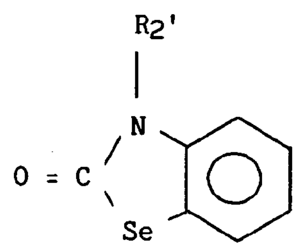

(II')

dans laquelle $R'_2$ représente un groupement alkyle inférieur,
dérivé de formule (II) ou (II'),
que l'on soumet, en fonction de la nature du substituant B du dérivé de formule (I) que l'on souhaite obtenir :

* ou bien à l'action d'un acide de formule (III) :

**B COOH (III)**

dans laquelle B a la même définition que dans la formule (I),
en présence d'acide polyphosphorique,

* ou bien à l'action d'un chlorure d'acide de formule (IV) :

**Cl COB (IV)**

dans laquelle B a la même définition que dans la formule (I),
ou encore à l'action d'un anhydride d'acide de formule (V) :

**$O(COB)_2$ (V)**

dans laquelle B a la même définition que dans la formule (I),
en présence de diméthylformamide et de chlorure d'aluminium,
pour obtenir un dérivé de formule (I/A) :

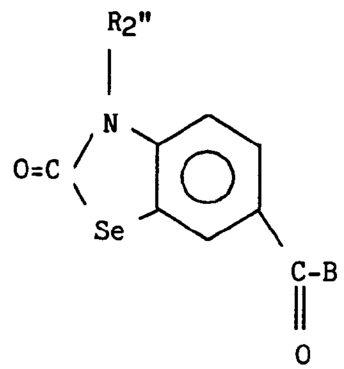

(I/A)

cas particulier des dérivés de formule (I) dans laquelle $R''_2$ représente un atome d'hydrogène ou un groupement alkyle inférieur, B a la même signification que dans la formule (I), A représente un groupement CO, et $R_1$ forme avec $R_4$ un groupement C=O,
que l'on purifie si nécessaire,
que l'on sépare, le cas échéant, en ses isomères et que l'on salifie le cas échéant, si on le désire, par une base pharmaceutiquement acceptable,
dérivé (I/A) que l'on peut, si on le désire, soumettre à l'action d'hydrure mixte de métal alcalin pour conduire à un dérivé de formule (I/B) :

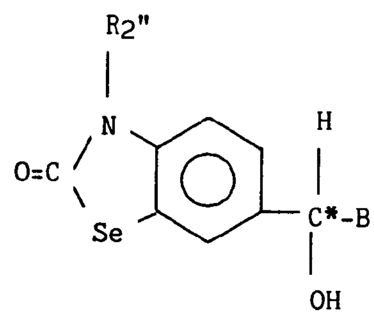

(I/B)

cas particulier des dérivés de formule (I), dans laquelle $R_2''$ a la même signification que précédemment, B a la même signification que dans la formule (I), A représente un groupement CHOH et $R_1$ forme avec $R_4$ un groupement CO,
que l'on purifie si nécessaire,
que l'on sépare, si on le désire en ses isomères et que l'on salifie le cas échéant par une base pharmaceutiquement acceptable,
dérivé de formule (I/A) que l'on peut encore, si on le désire, soumettre à l'action d'un trialkylsilane en milieu acide,
pour conduire à un dérivé de formule (I/C) :

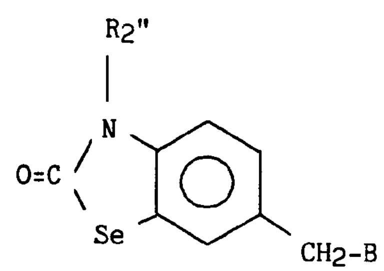

(I/C)

cas particulier des dérivés de formule (I) dans laquelle $R_2''$ et B ont la même signification que précédemment, A représente un groupement $CH_2$ et $R_1$ forme avec $R_4$ un groupement CO, que l'on purifie si nécessaire,
que l'on sépare, le cas échéant, en ses isomères et que l'on salifie le cas échéant par une base pharmaceutiquement acceptable,

dérivé de formule (I/A), (I/B) ou (I/C) que l'on peut, lorsque $R_2''$ représente un atome d'hydrogène, traiter :

- ou bien par un agent d'alkylation pour obtenir un dérivé de formule (I/D) :

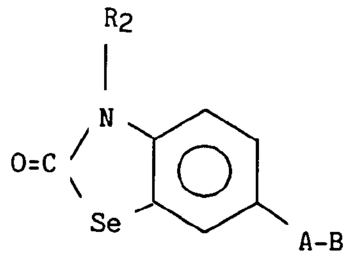

(I/D)

cas particulier des dérivés de formule (I) dans lesquelles A et B ont la même signification que dans la formule (I), $R_2$ représente un groupement alkyle inférieur linéaire ou ramifié,

- ou bien par un agent alcalin puis par un dérivé de formule (VI) :

$$X_1\text{-}M\text{-}X_2 \qquad (VI)$$

dans laquelle $X_1$ et $X_2$ différents représentent chacun un atome d'halogène, M représente un groupement alkyle inférieur linéaire ou ramifié pour conduire à un dérivé de formule (I/E) :

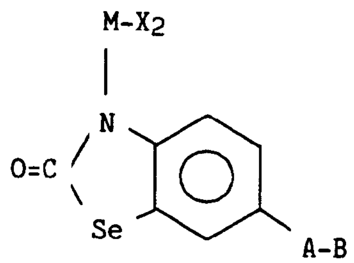

(I/E)

dans laquelle A, B, $X_2$ et M ont la même définition que précédemment, cas particulier des dérivés de formule (I) pour lesquels $R_2$ représente un groupement halogéno alkyle inférieur et $R_1$ forme avec $R_4$ un groupement CO, que l'on purifie, si nécessaire, et que l'on condense, si on le désire, avec une amine de formule (VIII) :

$$NR_5R_6 \qquad (VIII)$$

dans laquelle $R_5$ et $R_6$ ont la même définition que dans la formule (I),
pour obtenir un produit de formule (I/F) :

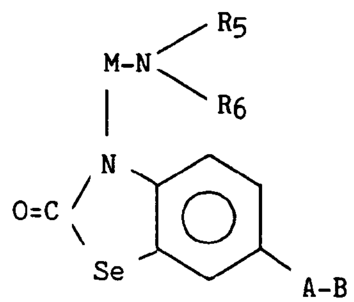

(I/F)

cas particulier des dérivés de formule (I) dans laquelle A et B ont la même signification que dans la formule (I), et $R_2$ représente un groupement alkyle inférieur substitué par un groupement $NR_5R_6$

- ou bien après action d'un agent alcalin par un dérivé de formule (IX) :

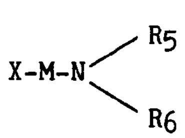

(IX)

dans laquelle X représente un atome d'halogène et M, $R_5$, $R_6$ ont la même signification que précédemment, pour conduire à un dérivé de formule (I/F) que l'on purifie si nécessaire quelque soit le procédé selon lequel il a été obtenu, que l'on sépare, le cas échéant en ses isomères et que l'on salifie, si on le désire par un acide pharmaceutiquement acceptable ou le cas échéant par une base pharmaceutiquement acceptable, dérivé de formule (I/F) qui, lorsque A représente un groupement CO, peut être soumis, si on le désire à l'action d'un hydrure mixte de métal alcalin pour conduire à un dérivé de formule (I/F1) :

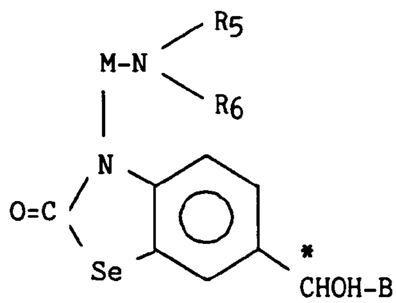

(I/F1)

dans laquelle A représente un groupement CHOH, M, $R_6$, $R_6$ et B ayant la même signification que précédemment, cas particulier des dérivés de formule (I) pour lesquels A représente un groupement CHOH, $R_2$ un groupement alkyle inférieur substitué par un groupement $NR_6R_6$ et $R_1$ forme avec $R_4$ un groupement CO,
que l'on purifie si nécessaire, que l'on sépare en ses isomères, si on le souhaite et que l'on salifie si on le désire par un acide pharmaceutiquement acceptable ou le cas échéant par une base pharmaceutiquement acceptable,
- dérivé de formule (I/A), (I/B), (I/C) ou (I/D) que l'on traite, si on le désire :
. ou bien par un hydroxyde de métal alcalin pour conduire à un dérivé de formule (I/G) :

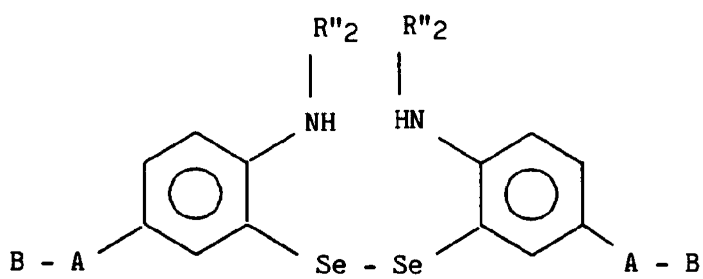

(I/G)

dans laquelle A, B, et $R''_2$ ont la même définition que précédemment, cas particulier des dérivés de formule (I),
. ou bien par une amine de formule $NR_7R_6$ en présence de formaldéhyde pour conduire à un dérivé de formule (I/H) :

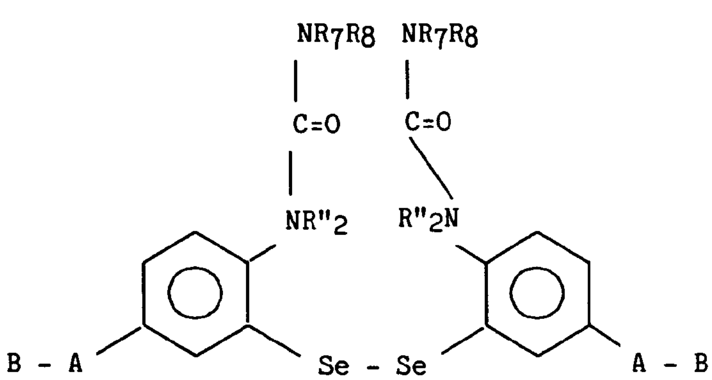

(I/H)

dans laquelle A, B, $R''_2$, $R_7$ et $R_8$ ont la même définition que précédemment, cas particulier des dérivés de formule (I),

dérivé de formule (I/G) ou (I/H) que l'on purifie, si on le désire par une technique classique de chromatographie et/ou cristallisation, que l'on peut le cas échéant séparer en ses isomères, et si on le désire, salifier le cas échéant par une base ou un acide pharmaceutiquement acceptable.

**20.** Procédé de préparation des dérivés de formule (I) selon la revendication 1 pour lesquels $R_2$ représente un chaînon méthyle substitué par un groupement $NR_5R_6$ ou un groupement OH et $R_1$ forme avec $R_4$ un groupement C=O caractérisé en ce que l'on place en milieu d'alcool aliphatique inférieur un dérivé de formule (II) ou (I/A), ou (I/B), ou (I/C) selon le dérivé de formule (I) que l'on souhaite obtenir :

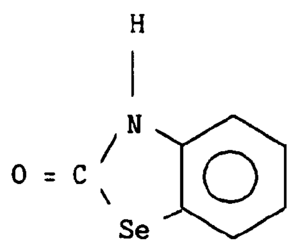

(II)

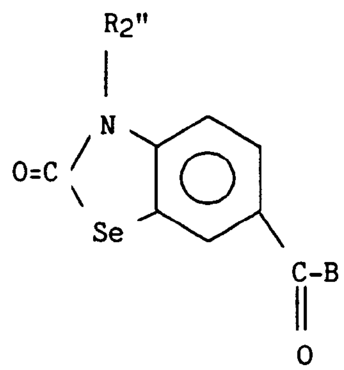

(I/A)

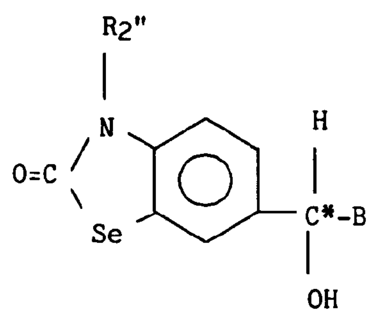

(I/B)

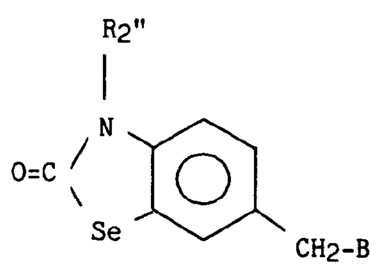

(I/C)

pour lesquels $R''_2$ représente un atome d'hydrogène et addition d'un excès de formol, chauffage de la solution obtenue à une température comprise entre la température ambiante et la température d'ébullition de la solution, pour conduire, après éventuel refroidissement, repos d'une à deux heures, filtration et éventuelle purification par chromatographie et/ou cristallisation à un dérivé de formule (I/J) :

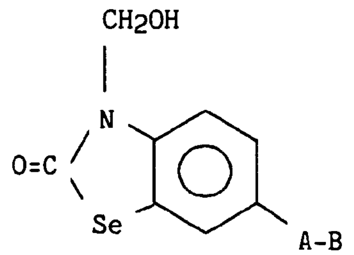

(I/J)

cas particulier des dérivés de formule (I) pour lesquels $R_2$ représente un groupement $CH_2OH$ et $R_1$ forme avec $R_4$ un groupement CO et A et B ont la même définition que dans la formule (I),
que l'on peut traiter par un agent d'halogénation pour conduire à un dérivé de formule (I/E1) :

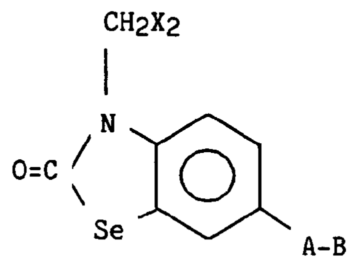

(I/E1)

dans lequel $X_2$ représente un atome d'halogène et A et B ont la même définition que dans la formule (I), cas particulier des dérivés de formule (I/E) pour lequel M représente un groupement $CH_2$, que l'on peut traiter par une amine de formule (VIII) :

**$NR_5R_6$ (VIII)**

dans laquelle $R_5$ et $R_6$ ont la même définition que dans la formule (I),
pour conduire à un dérivé de formle (I/F1) :

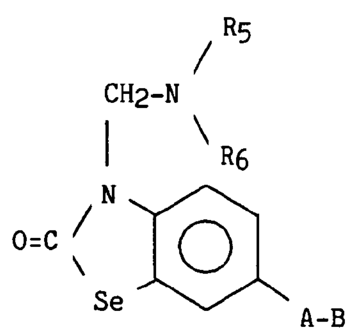

(I/F1)

cas particulier des dérivés de formule (I/F) dans laquelle M représente un groupement $CH_2$ et A, B, $R_6$ et $R_6$ ont la même définition que dans la formule (I),
que l'on purifie, si nécessaire, dont on sépare - le cas échéant les isomères, et que l'on salifie, si on le désire, par un acide ou le cas échéant une base pharmaceutiquement acceptable.

**21.** Procédé de préparation de dérivés de formule (I) selon la revendication 1 pour lesquels B représente un groupement phényle vinyle ou un groupement phényle vinyle substitué, A représente un groupement CO et $R_1$ forme avec $R_4$ un groupement CO caractérisé en ce que l'on fait réagir un dérivé de formule (I/I) :

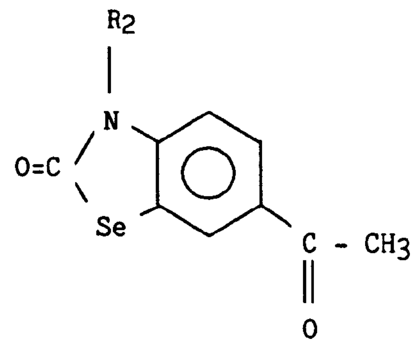

(I/I)

dans lequel $R_2$ a la même définition que dans la formule (I), avec un aldéhyde benzoïque de formule (X) :

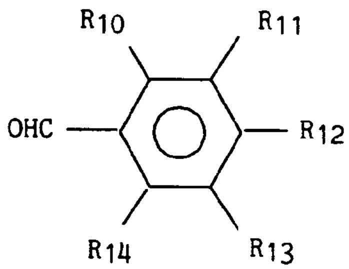

(X)

dans laquelle $R_{10}$, $R_{11}$, $R_{12}$, $R_{13}$, $R_{14}$ identiques ou différents représentent indépendamment l'un de l'autre un atome d'hydrogène, un groupement alkyle inférieur, alkoxy inférieur, hydroxy, trifluorométhyle, carboxy, amino ou atome d'halogène,
pour conduire à un dérivé de formule (I/S) :

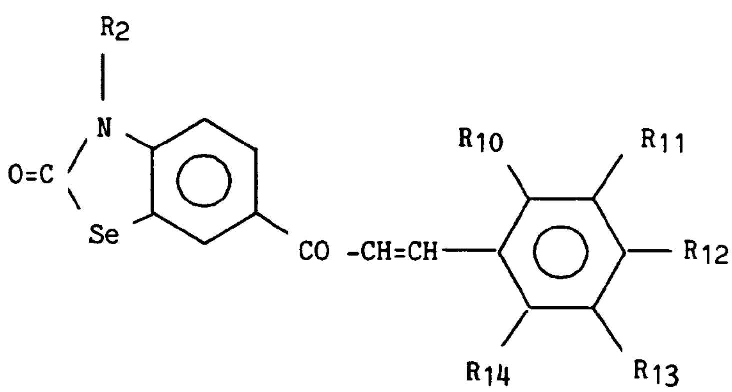

(I/S)

dans laquelle $R_2$, $R_{10}$, $R_{11}$, $R_{12}$, $R_{13}$, $R_{14}$ ont la même définition que précédemment,
que l'on purifie par une technique de chromatographie ou cristallisation dont on sépare les isomères, si on le désire, et que l'on salifie le cas échéant par une base ou un acide pharmaceutiquement acceptable.

**22.** Composition pharmaceutique contenant comme principe actif au moins un composé selon l'une des revendications 1 à 18 en combinaison avec un ou plusieurs excipients ou véhicules inertes, non toxiques, pharmaceutiquement acceptables.

**23.** Composition pharmaceutique selon la revendication 22 contenant au moins un principe actif selon l'une des revendications 1 à 18 utilisable dans le traitement des troubles inflammatoires et plus particulièrement dans les troubles rhumatismaux, les hyperlipidémies, le diabète non insulino dépendant et ses complications, dans la prévention et le traitement des accidents ischémiques artériels périphériques et cérébro vasculaires, dans le traitement de certains cancers ainsi qu'en tant que conservateur de certains organes.

**Revendications pour l'Etat contractant suivant: ES**

1. Procédé de préparation de dérivés de formule générale (I) :

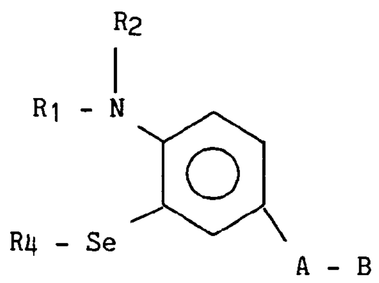

(I)

dans laquelle :

– $R_1$ représente soit un atome d'hydrogène soit un groupement $CO-NR_7R_8$ avec $R_7$, $R_8$ identiques ou différents, représentant un atome d'hydrogène, un groupement alkyle inférieur ou cycloalkyle de 3 à 8 atomes de carbone, ou aryle, ou aryle substitué, ou arylalkyle inférieur, ou arylalkyle inférieur substitué ou forment avec l'azote qui les porte un système hétérocyclique,

– $R_4$ représente un groupement :

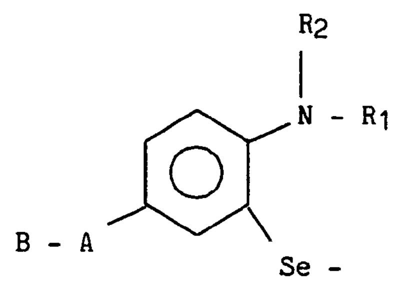

ou bien :

$R_4$ forme avec $R_1$ un groupement -C = O,

– $R_2$ représente un atome d'hydrogène ou un groupement alkyle inférieur éventuellement substitué par un ou plusieurs atomes d'halogène ou par un groupement hydroxy, ou lorsque $R_1$ forme avec $R_4$ un groupement CO, par un groupement $NR_8R_8$, dans laquelle $R_8$ et $R_6$, identiques ou différents représentent chacun indépendamment l'un de l'autre un atome d'hydrogène, un groupement alkyle inférieur, un groupement aryle, un groupement aryle substitué, un groupement arylalkyle inférieur, un groupement arylalkyle inférieur substitué,

ou bien $R_8$ et $R_8$ forment avec l'atome d'azote qui les porte un système hétérocyclique,

– A représente un groupement CO, CHOH ou $CH_2$,

– B représente un atome d'hydrogène, un groupement alkyle inférieur, un groupement cycloalkyle de 3 à 8 atomes de carbone, un groupement alcényle inférieur, un groupement alcynyle inférieur, un groupement aryle ou un groupement aryle substitué, un groupement hétéroaryle ou un groupement hétéroaryle substitué, un groupement arylalkyle inférieur, un groupement hétéroarylalkyle inférieur ou un groupement hétéroarylalkyle inférieur substitué, un groupement styryle, ou un groupement styryle substitué,

– ou A et B représentent ensemble un atome d'hydrogène à la condition que lorsque A et B forment ensemble un atome d'hydrogène :

. si $R_1$ forme avec $R_4$ un groupement CO alors $R_2$ ne peut représenter un groupement méthyle, ou un atome d'hydrogène,

. si $R_1$ ne forme pas avec $R_4$ un groupement CO alors $R_1$ et $R_2$ ne peuvent simultanément représenter un atome d'hydrogène,

étant entendu que :

– le terme hétérocyclique que l'on trouve dans les définitions de $R_1$ et $R_2$ représente un système mono ou bicyclique, chaque cycle comprenant de cinq à six sommets et incluant dans son squelette carboné un ou éventuellement plusieurs hétéroatomes identiques ou différents choisis parmi azote, oxygène ou soufre et éventuellement substitué par un groupement alkyle inférieur, par un groupement aryle, par

un groupement aryle substitué par un groupement arylalkyle, par un groupement arylalkyle substitué ; par un groupement hétéroaryle par un groupement hétéroaryle substitué ; par un ou plusieurs atomes d'halogène, par un groupement alkoxy inférieur,

– le terme substitué affectant les groupements aryle, arylalkyle inférieur, hétéroaryle, styryle dans les définitions de $R_1$, $R_2$ et de B signifie que ceux-ci sont substitués sur la partie aromatique par un ou plusieurs groupements identiques ou différents choisis parmi alkyle inférieur, alkoxy inférieur, hydroxy, trifluorométhyle, amino, carboxyle ou par un ou plusieurs atomes d'halogène,

– par groupement alkyle inférieur, alkoxy inférieur, alkényle inférieur, alcynyle inférieur on entend des groupements linéaires ou ramifiés comprenant de 1 à 6 atomes de carbone,

– par groupement aryle on entend un groupement choisi parmi phényle ou naphtyle,

– par groupement hétéroaryle, on entend un groupement aromatique mono ou bicyclique, chaque cycle comprenant cinq ou six sommets, l'ensemble des deux cycles incluant dans son squelette carboné de un à trois hétéroatomes choisis parmi azote, oxygène ou soufre,

leurs isomères, épimères, diastéréoisomères ainsi que lorsque B comprend un groupement carboxyle ou hydroxyle phénolique ou lorsque $R_1$ représente un atome d'hydrogène leurs sels d'addition à une base pharmaceutiquement acceptable, ainsi que lorsque $R_2$ représente un groupement amine, leurs sels d'addition à un acide pharmaceutiquement acceptable,
caractérisé en ce que l'on utilise comme matière première un dérivé de formule (II) :

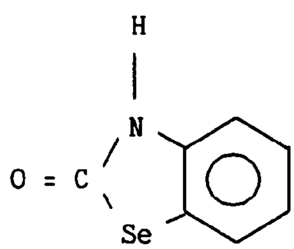

(II)

que l'on peut en fonction de la nature de $R_2$ du produit de formule (I) que l'on souhaite obtenir, alkyler à l'azote par un agent d'alkylation pour conduire à un dérivé de formule (II') :

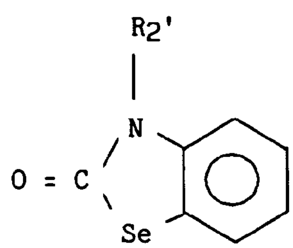

(II')

dans laquelle $R'_2$ représente un groupement alkyle inférieur,
dérivé de formule (II) ou (II'),
que l'on soumet, en fonction de la nature du substituant B du dérivé de formule (I) que l'on souhaite obtenir :

* ou bien à l'action d'un acide de formule (III) :

**B COOH (III)**

dans laquelle B a la même définition que dans la formule (I),
en présence d'acide polyphosphorique,

* ou bien à l'action d'un chlorure d'acide de formule (IV) :

**Cl COB (IV)**

dans laquelle B a la même définition que dans la formule (I),
ou encore à l'action d'un anhydride d'acide de formule (V) :

**O(COB)2 (V)**

dans laquelle B a la même définition que dans la formule (I),
en présence de diméthylformamide et de chlorure d'aluminium,
pour obtenir un dérivé de formule (I/A) :

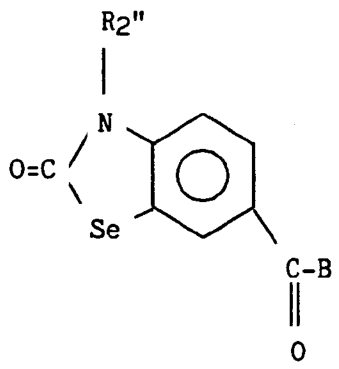

(I/A)

cas particulier des dérivés de formule (I) dans laquelle $R''_2$ représente un atome d'hydrogène ou un groupement alkyle inférieur, B a la même signification que dans la formule (I), A représente un groupement CO, et $R_1$ forme avec $R_4$ un groupement C=O,
que l'on purifie si nécessaire,
que l'on sépare, le cas échéant, en ses isomères et que l'on salifie le cas échéant, si on le désire, par une base pharmaceutiquement acceptable,
dérivé (I/A) que l'on peut, si on le désire, soumettre à l'action d'hydrure mixte de métal alcalin pour conduire à un dérivé de formule (I/B) :

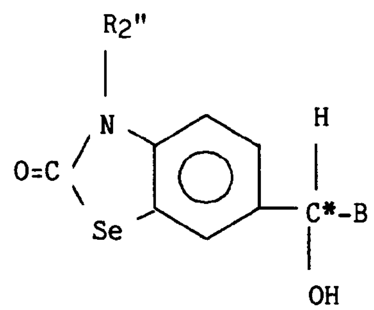

(I/B)

cas particulier des dérivés de formule (I), dans laquelle $R_2''$ a la même signification que précédemment, B a la même signification que dans la formule (I), A représente un groupement CHOH et $R_1$ forme avec $R_4$ un groupement CO,
que l'on purifie si nécessaire,
que l'on sépare, si on le désire en ses isomères et que l'on salifie le cas échéant par une base pharmaceutiquement acceptable,
dérivé de formule (I/A) que l'on peut encore, si on le désire, soumettre à l'action d'un trialkylsilane en milieu acide,
pour conduire à un dérivé de formule (I/C) :

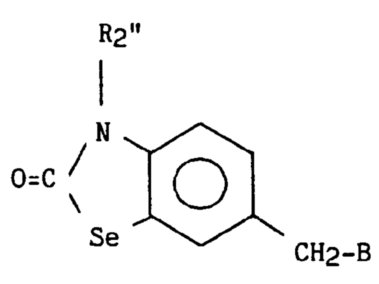

(I/C)

cas particulier des dérivés de formule (I) dans laquelle $R_2''$ et B ont la même signification que précédemment, A représente un groupement $CH_2$ et $R_1$ forme avec $R_4$ un groupement CO, que l'on purifie si nécessaire,
que l'on sépare, le cas échéant, en ses isomères et que l'on salifie le cas échéant par une base pharmaceutiquement acceptable,

dérivé de formule (I/A), (I/B) ou (I/C) que l'on peut, lorsque $R_2''$ représente un atome d'hydrogène, traiter :

- ou bien par un agent d'alkylation pour obtenir un dérivé de formule (I/D) :

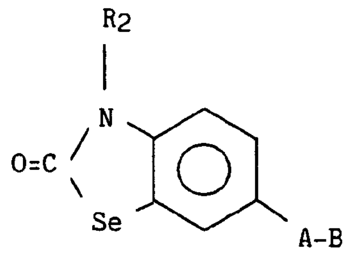

(I/D)

cas particulier des dérivés de formule (I) dans lesquelles A et B ont la même signification que dans la formule (I), $R_2$ représente un groupement alkyle inférieur linéaire ou ramifié,

- ou bien par un agent alcalin puis par un dérivé de formule (VI) :

$$X_1\text{-}M\text{-}X_2 \qquad (VI)$$

dans laquelle $X_1$ et $X_2$ différents représentent chacun un atome d'halogène, M représente un groupement alkyle inférieur linéaire ou ramifié pour conduire à un dérivé de formule (I/E) :

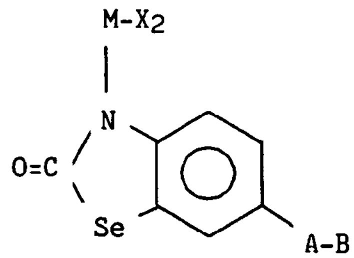

(I/E)

dans laquelle A, B, $X_2$ et M ont la même définition que précédemment, cas particulier des dérivés de formule (I) pour lesquels $R_2$ représente un groupement halogéno alkyle inférieur et $R_1$ forme avec $R_4$ un groupement CO,

que l'on purifie, si nécessaire, et que l'on condense, si on le désire, avec une amine de formule (VIII) :

$$NR_5R_6 \qquad (VIII)$$

dans laquelle $R_5$ et $R_6$ ont la même définition que dans la formule (I),

pour obtenir un produit de formule (I/F) :

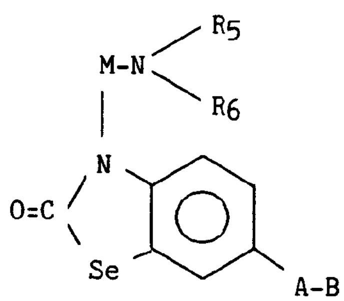

(I/F)

cas particulier des dérivés de formule (I) dans laquelle A et B ont la même signification que dans la formule (I), et $R_2$ représente un groupement alkyle inférieur substitué par un groupement $NR_5R_6$

- ou bien après action d'un agent alcalin par un dérivé de formule (IX) :

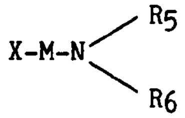

(IX)

dans laquelle X représente un atome d'halogène et M, $R_5$, $R_6$ ont la même signification que précédemment, pour conduire à un dérivé de formule (I/F) que l'on purifie si nécessaire quelque soit le procédé selon lequel il a été obtenu, que l'on sépare, le cas échéant en ses isomères et que l'on salifie, si on le désire par un acide pharmaceutiquement acceptable ou le cas échéant par une base pharmaceutiquement acceptable, dérivé de formule (I/F) qui, lorsque A représente un groupement CO, peut être soumis, si on le désire à l'action d'un hydrure mixte de métal alcalin pour conduire à un dérivé de formule (I/F1) :

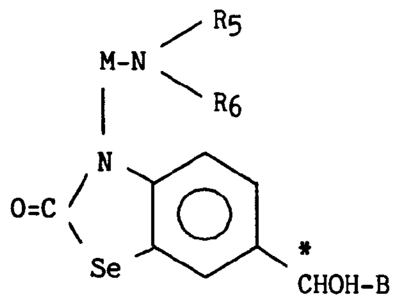

(I/F1)

dans laquelle A représente un groupement CHOH, M, $R_6$, $R_6$ et B ayant la même signification que précédemment, cas particulier des dérivés de formule (I) pour lesquels A représente un groupement CHOH, $R_2$ un groupement alkyle inférieur substitué par un groupement $NR_6R_6$ et $R_1$ forme avec $R_4$ un groupement CO,

que l'on purifie si nécessaire, que l'on sépare en ses isomères, si on le souhaite et que l'on salifie si on le désire par un acide pharmaceutiquement acceptable ou le cas échéant par une base pharmaceutiquement acceptable,

- dérivé de formule (I/A), (I/B), (I/C) ou (I/D) que l'on traite, si on le désire :

. ou bien par un hydroxyde de métal alcalin pour conduire à un dérivé de formule (I/G) :

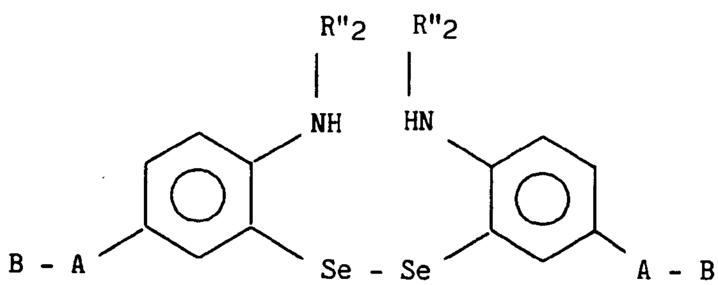

(I/G)

dans laquelle A, B, et $R''_2$ ont la même définition que précédemment, cas particulier des dérivés de formule (I),

. ou bien par une amine de formule $NR_7R_6$ en présence de formaldéhyde pour conduire à un dérivé de formule (I/H) :

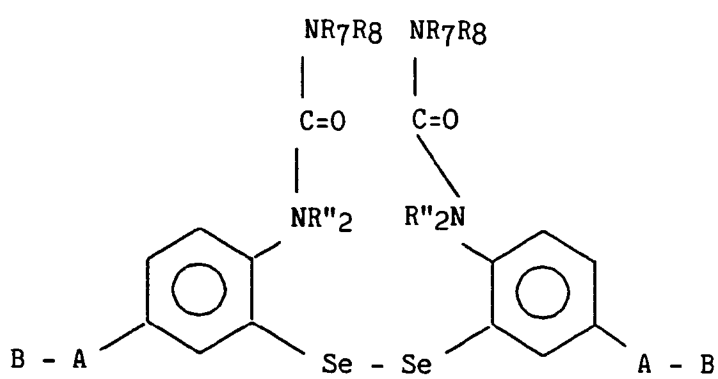

(I/H)

dans laquelle A, B, $R''_2$, $R_7$ et $R_8$ ont la même définition que précédemment, cas particulier des dérivés de formule (I),
dérivé de formule (I/G) ou (I/H) que l'on purifie, si on le désire par une technique classique de chromatographie et/ou cristallisation, que l'on peut le cas échéant séparer en ses isomères, et si on le désire, salifier le cas échéant par une base ou un acide pharmaceutiquement acceptable.

**2.** Procédé de préparation des dérivés de formule (I) selon la revendication 1 pour lesquels $R_2$ représente un chaînon méthyle substitué par un groupement $NR_5R_6$ ou un groupement OH et $R_1$ forme avec $R_4$ un groupement C=O caractérisé en ce que l'on place en milieu d'alcool aliphatique inférieur un dérivé de formule (II) ou (I/A), ou (I/B), ou (I/C) selon le dérivé de formule (I) que l'on souhaite obtenir :

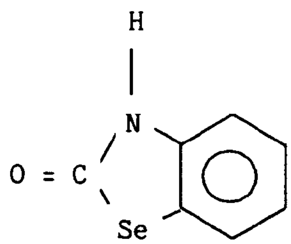

(II)

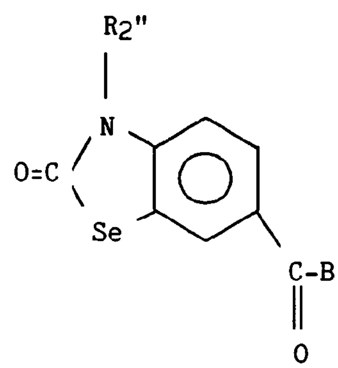

(I/A)

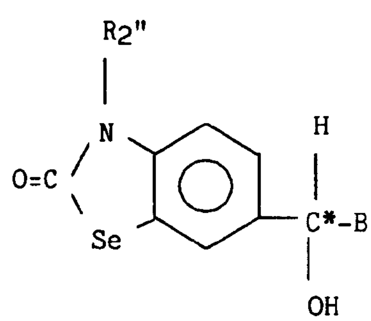

(I/B)

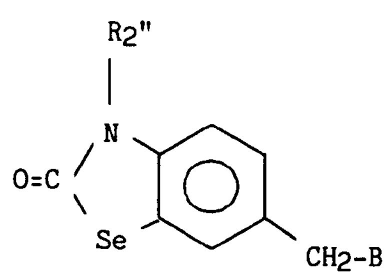

(I/C)

pour lesquels $R''_2$ représente un atome d'hydrogène et addition d'un excès de formol, chauffage de la solution obtenue à une température comprise entre la température ambiante et la température d'ébullition de la solution, pour conduire, après éventuel refroidissement, repos d'une à deux heures, filtration et éventuelle purification par chromatographie et/ou cristallisation à un dérivé de formule (I/J) :

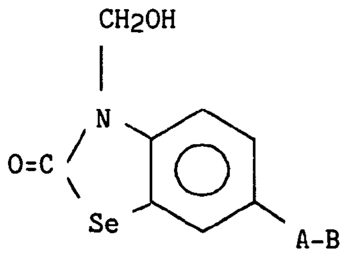

(I/J)

cas particulier des dérivés de formule (I) pour lesquels $R_2$ représente un groupement $CH_2OH$ et $R_1$ forme avec $R_4$ un groupement CO et A et B ont la même définition que dans la formule (I), que l'on peut traiter par un agent d'halogénation pour conduire à un dérivé de formule (I/E1) :

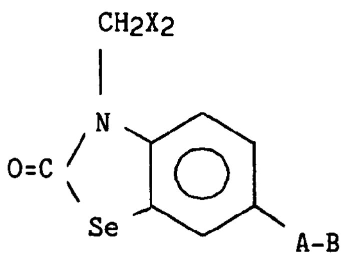

(I/E1)

dans lequel $X_2$ représente un atome d'halogène et A et B ont la même définition que dans la formule (I), cas particulier des dérivés de formule (I/E) pour lequel M représente un groupement $CH_2$, que l'on peut traiter par une amine de formule (VIII) :

$$NR_5R_6 \qquad (VIII)$$

dans laquelle $R_5$ et $R_6$ ont la même définition que dans la formule (I), pour conduire à un dérivé de formle (I/F1) :

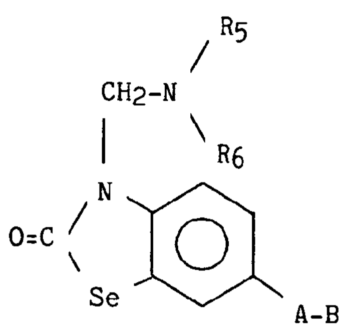

(I/F1)

cas particulier des dérivés de formule (I/F) dans laquelle M représente un groupement $CH_2$ et A, B, $R_6$ et $R_6$ ont la même définition que dans la formule (I), que l'on purifie, si nécessaire, dont on sépare - le cas échéant les isomères, et que l'on salifie, si on le désire, par un acide ou le cas échéant une base pharmaceutiquement acceptable.

**3.** Procédé de préparation de dérivés de formule (I) selon la revendication 1 pour lesquels B représente un groupement phényle vinyle ou un groupement phényle vinyle substitué, A représente un groupement CO et $R_1$ forme avec $R_4$ un groupement CO caractérisé en ce que l'on fait réagir un dérivé de formule (I/I) :

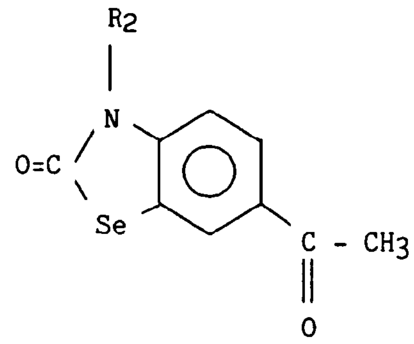

(I/I)

dans lequel $R_2$ a la même définition que dans la formule (I), avec un aldéhyde benzoïque de formule (X) :

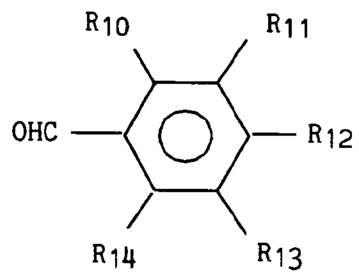

(X)

dans laquelle $R_{10}$, $R_{11}$, $R_{12}$, $R_{13}$, $R_{14}$ identiques ou différents représentent indépendamment l'un de l'autre un atome d'hydrogène, un groupement alkyle inférieur, alkoxy inférieur, hydroxy, trifluorométhyle, carboxy, amino ou atome d'halogène, pour conduire à un dérivé de formule (I/S) :

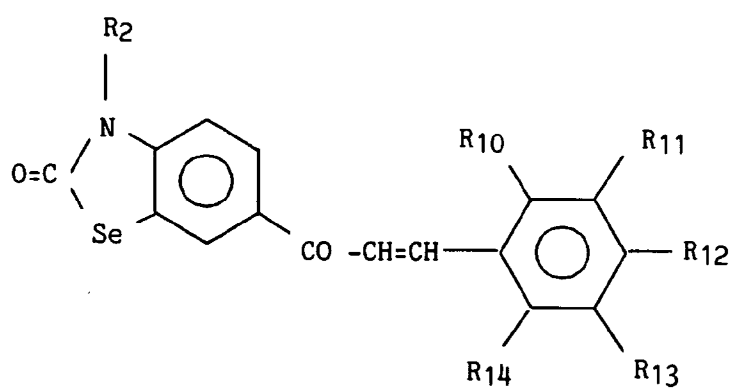

(I/S)

dans laquelle $R_2$, $R_{10}$, $R_{11}$, $R_{12}$, $R_{13}$, $R_{14}$ ont la même définition que précédemment, que l'on purifie par une technique de chromatographie ou cristallisation dont on sépare les isomères, si on le désire, et que l'on salifie le cas échéant par une base ou un acide pharmaceutiquement acceptable.

**4.** Procédé de préparation selon la revendication 1 de composés pour lesquels $R_1$ forme avec $R_4$ un groupement CO, de formule (I/D1) :

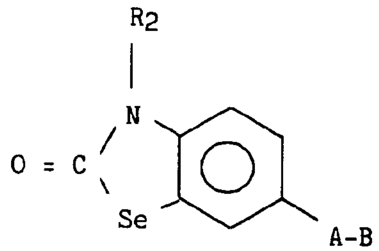

(I/D1)

pour lesquelles $R_2$, A et B ont la même définition que dans la formule (I), leurs isomères ainsi que le cas échéant, leurs sels d'addition à un acide ou une base pharmaceutiquement acceptables.

**5.** Procédé de préparation selon la revendication 1 de composés de formule (I/G1) :

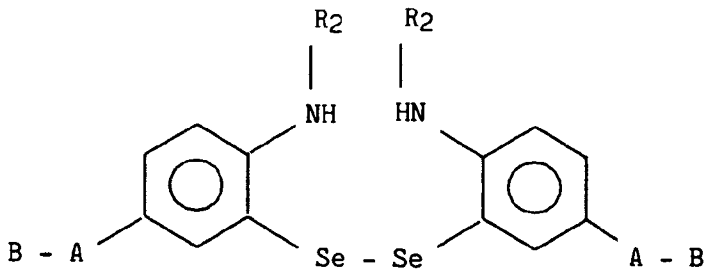

(I/G1)

dans laquelle A, B et R2 ont la même définition que dans la formule (I), leurs isomères ainsi que le cas échéant, leurs sels d'addition à un acide ou une base pharmaceutiquement acceptables.

**6.** Procédé de préparation selon la revendication 1 de composés pour lesquels A-B représentent un atome d'hydrogène, leurs isomères ainsi que le cas échéant, leurs sels d'addition à un acide ou une base pharmaceutiquement acceptable.

**7.** Procédé de préparation selon la revendication 1 de composés de formule générale (I/F) :

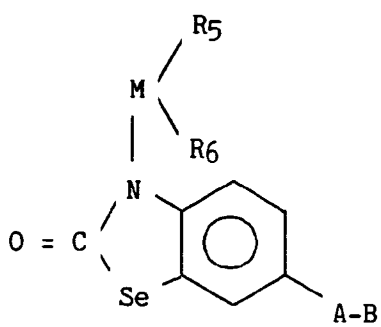

(I/F)

pour lesquels M représente un chaînon alkyle inférieur et A, B, $R_5$ et $R_5$ ont la même définition que dans la formule (I), leurs isomères ainsi que leurs sels d'addition à un acide ou une base pharmaceutiquement acceptables.

**8.** Procédé de préparation selon la revendication 1 de composés de formule (I/S) :

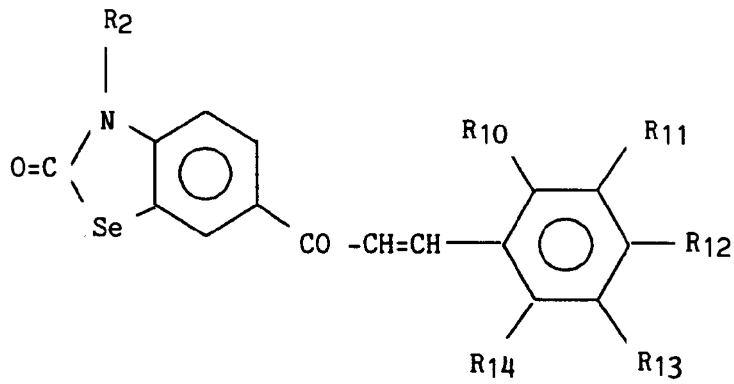

(I/S)

dans laquelle $R_2$ a la même signification que dans la revendication 1 et $R_{10}$, $R_{11}$, $R_{12}$, $R_{13}$ et $R_{14}$ identiques ou différents représentent indépendamment l'un de l'autre un atome d'hydrogène, un groupement alkyle inférieur, alkoxy inférieur, hydroxy, trifluorométhyle, amino ou atome d'halogène, leurs isomères ainsi que le cas échéant, leurs sels d'addition à un acide ou une base pharmaceutiquement acceptable.

**9.** Procédé de préparation selon la revendication 1 de composés pour lesquels A représente un groupement CO et B représente un groupement phényle, leurs isomères ainsi que le cas échéant, leurs sels d'addition à une base pharmaceutiquement acceptable.

**10.** Procédé de préparation selon la revendication 1 du composé qui est la 6-(3',5'-ditert.butyl 4'-hydroxy)cinnamoyl benzosélénazolinone ses isomères, ainsi que ses sels d'addition à une base pharmaceutiquement acceptable.

**11.** Procédé de préparation selon la revendication 1 du composé qui est la 3-méthyl 6-(3',5'-ditertbutyl 4'-hydroxy)cinnamoyl benzoselenazolinone, ses isomères, ainsi que ses sels d'addition à une base pharmaceutiquement acceptable.

**12.** Procédé de préparation selon la revendication 1 du composé qui est la 6-benzoyl benzosélénazolinone ainsi que ses sels d'addition à une base pharmaceutiquement acceptable.

**13.** Procédé de préparation selon la revendication 1 du composé qui est le bis [(2-amino 5-benzoyl) phényl] diséléniure ainsi que ses sels d'addition à un acide pharmaceutiquement acceptable.

**14.** Procédé de préparation selon la revendication 1 du composé qui est le bis [(2-méthyl amino 5-benzoyl) phényl] diséléniure ainsi que ses sels d'addition à un acide pharmaceutiquement acceptable.

**15.** Procédé de préparation selon la revendication 1 du composé qui est le bis [2-(3,3-diméthyl uréido) phényl] diséléniure.

**16.** Procédé de préparation selon la revendication 1 du composé qui est le bis [2-amino 5-(phényl hydroxy méthyl)phényl] diséléniure ainsi que ses sels d'addition à une base pharmaceutiquement acceptable.

**17.** Procédé de préparation selon la revendication 1 du composé qui est le bis [2-(3,3-diméthyl uréido)5-benzoyl phényl] diséléniure.

**18.** Procédé de préparation selon la revendication 1 du composé qui est le bis [2-amino 5-acétyl phényl] diséléniure ainsi que ses sels d'addition à un acide pharmaceutiquement acceptable.

**19.** Procédé de préparation selon la revendication 1 du composé qui est le bis [(2-méthyl amino) phényl]diséléniure ainsi que ses sels d'addition à un acide pharmaceutiquement acceptable.

**20.** Procédé de préparation selon la revendication 1 du composé qui est la 6-[(phényl) hydroxy méthyl] benzosélénazolinone ainsi que ses sels d'addition à une base pharmaceutiquement acceptable.

**21.** Procédé de préparation de composition pharmaceutique contenant comme principe actif au moins un composé obtenu selon l'une des revendications 1 à 20 en combinaison avec un ou plusieurs excipients ou véhicules inertes, non toxiques, pharmaceutiquement acceptables.

**22.** Procédé de préparation de composition pharmaceutique selon la revendication 21 contenant au moins un principe actif obtenu selon l'une des revendications 1 à 20 utilisables dans le traitement des troubles inflammatoires et plus particulièrement dans les troubles rhumatismaux, les hyperlipidémies, le diabète non insulino dépendant et ses complications, dans la prévention et le traitement des accidents ischémiques artériels périphériques et cérébro vasculaires, dans le traitement de certains cancers ainsi qu'en tant que conservateur de certains organes.

**Revendications pour l'Etat contractant suivant: GR**

**1.** Procédé de préparation de dérivés de formule générale (I) :

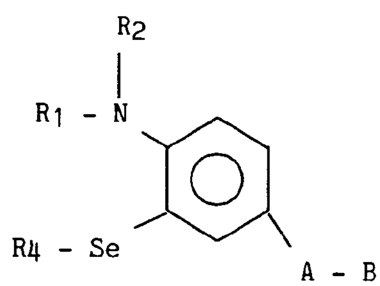

(I)

dans laquelle :

– $R_1$ représente soit un atome d'hydrogène soit un groupement CO-$NR_7R_8$ avec $R_7$, $R_8$ identiques ou différents, représentant un atome d'hydrogène, un groupement alkyle inférieur ou cycloalkyle de 3 à 8 atomes de carbone, ou aryle, ou aryle substitué, ou arylalkyle inférieur, ou arylalkyle inférieur substitué ou forment avec l'azote qui les porte un système hétérocyclique,

– $R_4$ représente un groupement :

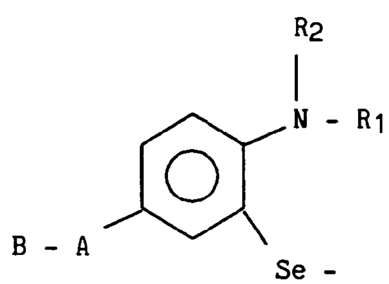

ou bien :

$R_4$ forme avec $R_1$ un groupement -C = O,

– $R_2$ représente un atome d'hydrogène ou un groupement alkyle inférieur éventuellement substitué par un ou plusieurs atomes d'halogène ou par un groupement hydroxy, ou lorsque $R_1$ forme avec $R_4$ un groupement CO, par un groupement $NR_8R_8$, dans laquelle $R_8$ et $R_6$, identiques ou différents représentent chacun indépendamment l'un de l'autre un atome d'hydrogène, un groupement alkyle inférieur, un groupement aryle, un groupement aryle substitué, un groupement arylalkyle inférieur, un groupement arylalkyle inférieur substitué,

ou bien $R_8$ et $R_8$ forment avec l'atome d'azote qui les porte un système hétérocyclique,

– A représente un groupement CO, CHOH ou $CH_2$,

– B représente un atome d'hydrogène, un groupement alkyle inférieur, un groupement cycloalkyle de 3 à 8 atomes de carbone, un groupement alcényle inférieur, un groupement alcynyle inférieur, un groupement aryle ou un groupement aryle substitué, un groupement hétéroaryle ou un groupement hétéroaryle substitué, un groupement arylalkyle inférieur, un groupement hétéroarylalkyle inférieur ou un

groupement hétéroarylalkyle inférieur substitué, un groupement styryle, ou un groupement styryle substitué,

– ou A et B représentent ensemble un atome d'hydrogène à la condition que lorsque A et B forment ensemble un atome d'hydrogène :

. si $R_1$ forme avec $R_4$ un groupement CO alors $R_2$ ne peut représenter un groupement méthyle, ou un atome d'hydrogène,

. si $R_1$ ne forme pas avec $R_4$ un groupement CO alors $R_1$ et $R_2$ ne peuvent simultanément représenter un atome d'hydrogène,

étant entendu que :

– le terme hétérocyclique que l'on trouve dans les définitions de $R_1$ et $R_2$ représente un système mono ou bicyclique, chaque cycle comprenant de cinq à six sommets et incluant dans son squelette carboné un ou éventuellement plusieurs hétéroatomes identiques ou différents choisis parmi azote, oxygène ou soufre et éventuellement substitué par un groupement alkyle inférieur, par un groupement aryle, par un groupement aryle substitué par un groupement arylalkyle, par un groupement arylalkyle substitué ; par un groupement hétéroaryle par un groupement hétéroaryle substitué ; par un ou plusieurs atomes d'halogène, par un groupement alkoxy inférieur,

– le terme substitué affectant les groupements aryle, arylalkyle inférieur, hétéroaryle, styryle dans les définitions de $R_1$, $R_2$ et de B signifie que ceux-ci sont substitués sur la partie aromatique par un ou plusieurs groupements identiques ou différents choisis parmi alkyle inférieur, alkoxy inférieur, hydroxy, trifluorométhyle, amino, carboxyle ou par un ou plusieurs atomes d'halogène,

– par groupement alkyle inférieur, alkoxy inférieur, alkényle inférieur, alcynyle inférieur on entend des groupements linéaires ou ramifiés comprenant de 1 à 6 atomes de carbone,

– par groupement aryle on entend un groupement choisi parmi phényle ou naphtyle,

– par groupement hétéroaryle, on entend un groupement aromatique mono ou bicyclique, chaque cycle comprenant cinq ou six sommets, l'ensemble des deux cycles incluant dans son squelette carboné de un à trois hétéroatomes choisis parmi azote, oxygène ou soufre,

leurs isomères, épimères, diastéréoisomères ainsi que lorsque B comprend un groupement carboxyle ou hydroxyle phénolique ou lorsque $R_1$ représente un atome d'hydrogène leurs sels d'addition à une base pharmaceutiquement acceptable, ainsi que lorsque $R_2$ représente un groupement amine, leurs sels d'addition à un acide pharmaceutiquement acceptable,

caractérisé en ce que l'on utilise comme matière première un dérivé de formule (II) :

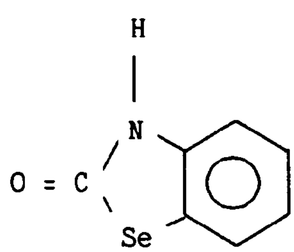

(II)

que l'on peut en fonction de la nature de $R_2$ du produit de formule (I) que l'on souhaite obtenir, alkyler à l'azote par un agent d'alkylation pour conduire à un dérivé de formule (II') :

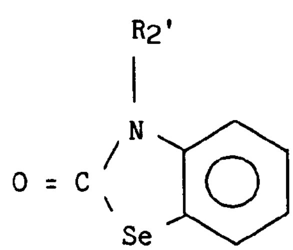

(II')

dans laquelle $R'_2$ représente un groupement alkyle inférieur,

dérivé de formule (II) ou (II'),

que l'on soumet, en fonction de la nature du substituant B du dérivé de formule (I) que l'on souhaite obtenir :

* ou bien à l'action d'un acide de formule (III) :

**B COOH (III)**

dans laquelle B a la même définition que dans la formule (I), en présence d'acide polyphosphorique,

* ou bien à l'action d'un chlorure d'acide de formule (IV) :

**Cl COB (IV)**

dans laquelle B a la même définition que dans la formule (I), ou encore à l'action d'un anhydride d'acide de formule (V) :

**O(COB)2 (V)**

dans laquelle B a la même définition que dans la formule (I), en présence de diméthylformamide et de chlorure d'aluminium, pour obtenir un dérivé de formule (I/A) :

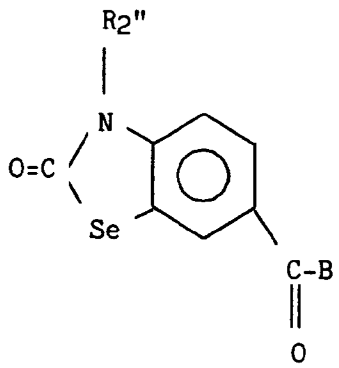

(I/A)

cas particulier des dérivés de formule (I) dans laquelle $R''_2$ représente un atome d'hydrogène ou un groupement alkyle inférieur, B a la même signification que dans la formule (I), A représente un groupement CO, et $R_1$ forme avec $R_4$ un groupement C=O,
que l'on purifie si nécessaire,
que l'on sépare, le cas échéant, en ses isomères et que l'on salifie le cas échéant, si on le désire, par une base pharmaceutiquement acceptable,
dérivé (I/A) que l'on peut, si on le désire, soumettre à l'action d'hydrure mixte de métal alcalin pour conduire à un dérivé de formule (I/B) :

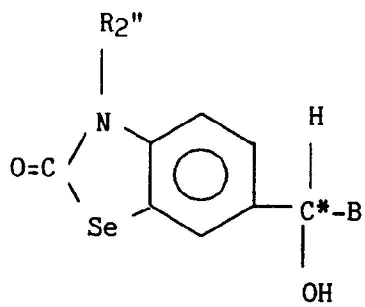

(I/B)

cas particulier des dérivés de formule (I), dans laquelle $R_2''$ a la même signification que précédemment, B a la même signification que dans la formule (I), A représente un groupement CHOH et $R_1$ forme avec $R_4$ un groupement CO,
que l'on purifie si nécessaire,
que l'on sépare, si on le désire en ses isomères et que l'on salifie le cas échéant par une base pharmaceutiquement acceptable,
dérivé de formule (I/A) que l'on peut encore, si on le désire, soumettre à l'action d'un trialkylsilane en milieu acide,
pour conduire à un dérivé de formule (I/C) :

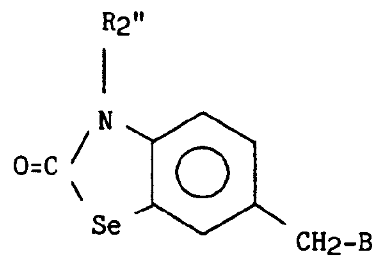

(I/C)

cas particulier des dérivés de formule (I) dans laquelle $R_2''$ et B ont la même signification que précédemment, A représente un groupement $CH_2$ et $R_1$ forme avec $R_4$ un groupement CO, que l'on purifie si nécessaire,
que l'on sépare, le cas échéant, en ses isomères et que l'on salifie le cas échéant par une base pharmaceutiquement acceptable,
dérivé de formule (I/A), (I/B) ou (I/C) que l'on peut, lorsque $R_2''$ représente un atome d'hydrogène, traiter :
- ou bien par un agent d'alkylation pour obtenir un dérivé de formule (I/D) :

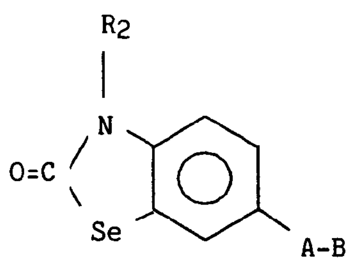

(I/D)

cas particulier des dérivés de formule (I) dans lesquelles A et B ont la même signification que dans la formule (I), $R_2$ représente un groupement alkyle inférieur linéaire ou ramifié,
- ou bien par un agent alcalin puis par un dérivé de formule (VI) :

$$X_1\text{-}M\text{-}X_2 \qquad (VI)$$

dans laquelle $X_1$ et $X_2$ différents représentent chacun un atome d'halogène, M représente un groupement alkyle inférieur linéaire ou ramifié pour conduire à un dérivé de formule (I/E) :

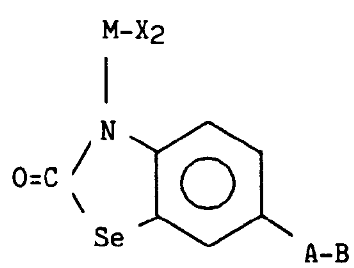

(I/E)

dans laquelle A, B, $X_2$ et M ont la même définition que précédemment, cas particulier des dérivés de formule (I) pour lesquels $R_2$ représente un groupement halogéno alkyle inférieur et $R_1$ forme avec $R_4$ un groupement CO,
que l'on purifie, si nécessaire, et que l'on condense, si on le désire, avec une amine de formule (VIII) :

$$NR_5R_6 \qquad (VIII)$$

dans laquelle $R_5$ et $R_6$ ont la même définition que dans la formule (I),
pour obtenir un produit de formule (I/F) :

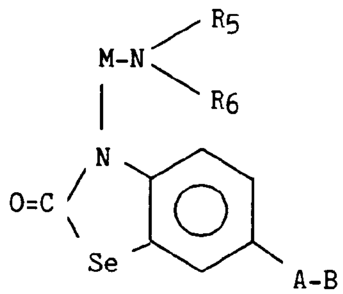

(I/F)

cas particulier des dérivés de formule (I) dans laquelle A et B ont la même signification que dans la formule (I), et $R_2$ représente un groupement alkyle inférieur substitué par un groupement $NR_5R_6$

- ou bien après action d'un agent alcalin par un dérivé de formule (IX) :

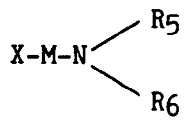

(IX)

dans laquelle X représente un atome d'halogène et M, $R_5$, $R_6$ ont la même signification que précédemment, pour conduire à un dérivé de formule (I/F) que l'on purifie si nécessaire quelque soit le procédé selon lequel il a été obtenu, que l'on sépare, le cas échéant en ses isomères et que l'on salifie, si on le désire par un acide pharmaceutiquement acceptable ou le cas échéant par une base pharmaceutiquement acceptable, dérivé de formule (I/F) qui, lorsque A représente un groupement CO, peut être soumis, si on le désire à l'action d'un hydrure mixte de métal alcalin pour conduire à un dérivé de formule (I/F1) :

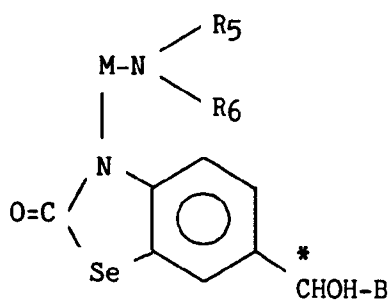

(I/F1)

dans laquelle A représente un groupement CHOH, M, $R_6$, $R_6$ et B ayant la même signification que précédemment, cas particulier des dérivés de formule (I) pour lesquels A représente un groupement CHOH, $R_2$ un groupement alkyle inférieur substitué par un groupement $NR_6R_6$ et $R_1$ forme avec $R_4$ un groupement CO,

que l'on purifie si nécessaire, que l'on sépare en ses isomères, si on le souhaite et que l'on salifie si on le désire par un acide pharmaceutiquement acceptable ou le cas échéant par une base pharmaceutiquement acceptable,

- dérivé de formule (I/A), (I/B), (I/C) ou (I/D) que l'on traite, si on le désire :

. ou bien par un hydroxyde de métal alcalin pour conduire à un dérivé de formule (I/G) :

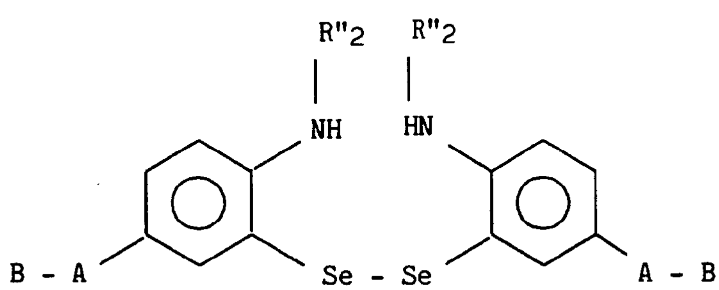

(I/G)

dans laquelle A, B, et $R''_2$ ont la même définition que précédemment, cas particulier des dérivés de formule (I),

. ou bien par une amine de formule $NR_7R_8$ en présence de formaldéhyde pour conduire à un dérivé de formule (I/H) :

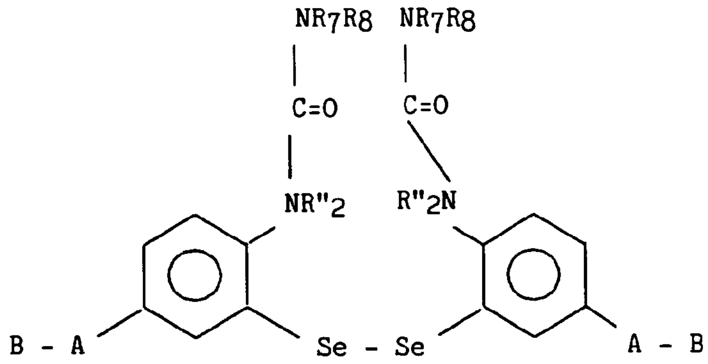

(I/H)

dans laquelle A, B, $R''_2$, $R_7$ et $R_8$ ont la même définition que précédemment, cas particulier des dérivés de formule (I),

dérivé de formule (I/G) ou (I/H) que l'on purifie, si on le désire par une technique classique de chromatographie et/ou cristallisation, que l'on peut le cas échéant séparer en ses isomères, et si on le désire, salifier le cas échéant par une base ou un acide pharmaceutiquement acceptable.

2. Procédé de préparation des dérivés de formule (I) selon la revendication 1 pour lesquels $R_2$ représente un chaînon méthyle substitué par un groupement $NR_5R_6$ ou un groupement OH et $R_1$ forme avec $R_4$ un groupement C=O caractérisé en ce que l'on place en milieu d'alcool aliphatique inférieur un dérivé de formule (II) ou (I/A), ou (I/B), ou (I/C) selon le dérivé de formule (I) que l'on souhaite obtenir :

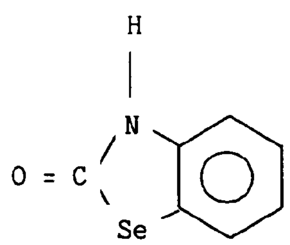

(II)

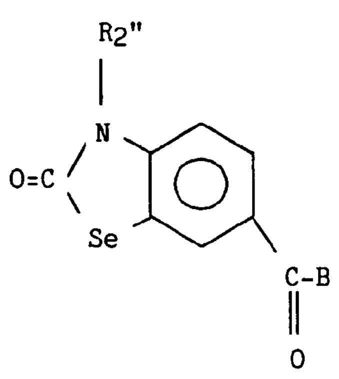

(I/A)

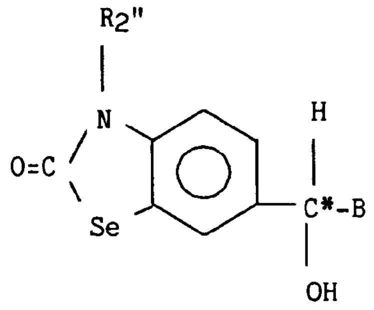

(I/B)

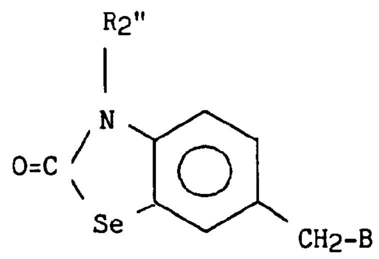

(I/C)

pour lesquels $R''_2$ représente un atome d'hydrogène et addition d'un excès de formol, chauffage de la solution obtenue à une température comprise entre la température ambiante et la température d'ébullition de la solution, pour conduire, après éventuel refroidissement, repos d'une à deux heures, filtration et éventuelle purification par chromatographie et/ou cristallisation à un dérivé de formule (I/J) :

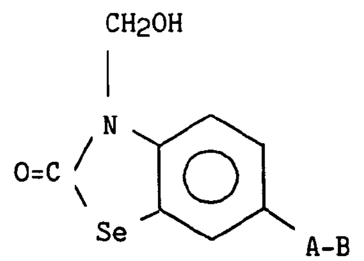

(I/J)

cas particulier des dérivés de formule (I) pour lesquels $R_2$ représente un groupement $CH_2OH$ et $R_1$ forme avec $R_4$ un groupement CO et A et B ont la même définition que dans la formule (I),
que l'on peut traiter par un agent d'halogénation pour conduire à un dérivé de formule (I/E1) :

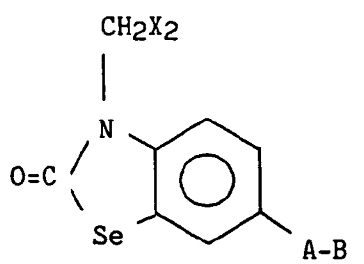

(I/E1)

dans lequel $X_2$ représente un atome d'halogène et A et B ont la même définition que dans la formule (I), cas particulier des dérivés de formule (I/E) pour lequel M représente un groupement $CH_2$, que l'on peut traiter par une amine de formule (VIII) :

**$NR_5R_6$ (VIII)**

dans laquelle $R_5$ et $R_6$ ont la même définition que dans la formule (I),
pour conduire à un dérivé de formle (I/F1) :

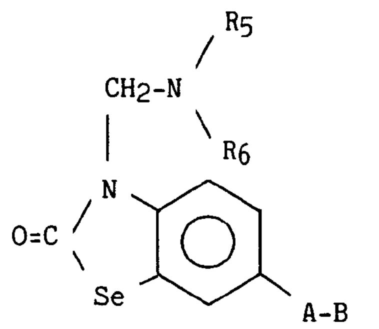

(I/F1)

cas particulier des dérivés de formule (I/F) dans laquelle M représente un groupement $CH_2$ et A, B, $R_5$ et $R_6$ ont la même définition que dans la formule (I), que l'on purifie, si nécessaire, dont on sépare - le cas échéant les isomères, et que l'on salifie, si on le désire, par un acide ou le cas échéant une base pharmaceutiquement acceptable.

**3.** Procédé de préparation de dérivés de formule (I) selon la revendication 1 pour lesquels B représente un groupement phényle vinyle ou un groupement phényle vinyle substitué, A représente un groupement CO et $R_1$ forme avec $R_4$ un groupement CO caractérisé en ce que l'on fait réagir un dérivé de formule (I/I) :

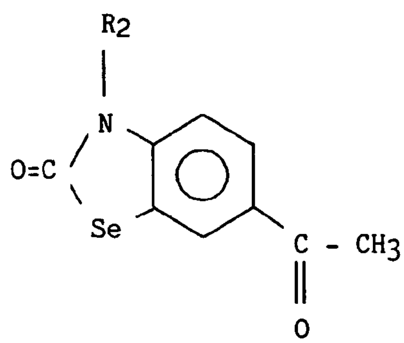

(I/I)

dans lequel $R_2$ a la même définition que dans la formule (I), avec un aldéhyde benzoïque de formule (X) :

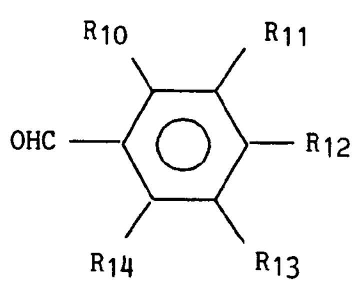

(X)

dans laquelle $R_{10}$, $R_{11}$, $R_{12}$, $R_{13}$, $R_{14}$ identiques ou différents représentent indépendamment l'un de l'autre un atome d'hydrogène, un groupement alkyle inférieur, alkoxy inférieur, hydroxy, trifluorométhyle, carboxy, amino ou atome d'halogène, pour conduire à un dérivé de formule (I/S) :

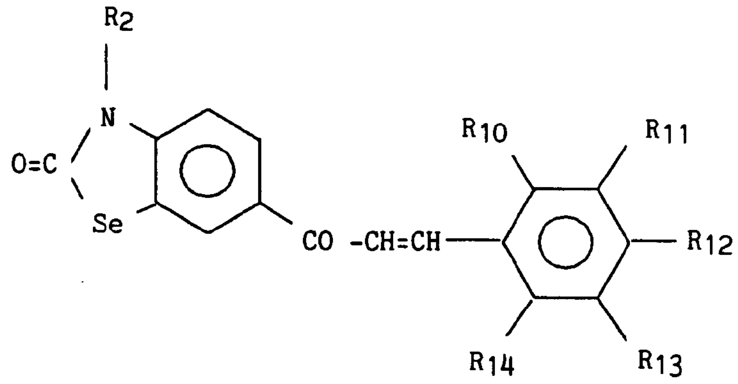

(I/S)

dans laquelle $R_2$, $R_{10}$, $R_{11}$, $R_{12}$, $R_{13}$, $R_{14}$ ont la même définition que précédemment, que l'on purifie par une technique de chromatographie ou cristallisation dont on sépare les isomères, si on le désire, et que l'on salifie le cas échéant par une base ou un acide pharmaceutiquement acceptable.

4. Procédé de préparation selon la revendication 1 de composés pour lesquels $R_1$ forme avec $R_4$ un groupement CO, de formule (I/D1) :

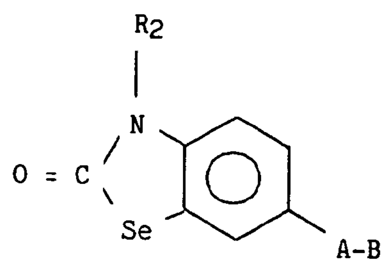

(I/D1)

pour lesquelles $R_2$, A et B ont la même définition que dans la formule (I), leurs isomères ainsi que le cas échéant, leurs sels d'addition à un acide ou une base pharmaceutiquement acceptables.

5. Procédé de préparation selon la revendication 1 de composés de formule (I/G1) :

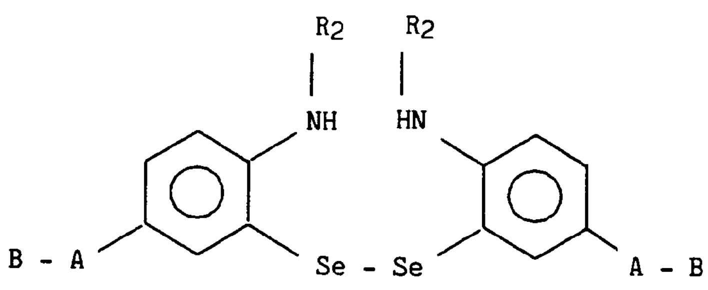

(I/G1)

dans laquelle A, B et R2 ont la même définition que dans la formule (I), leurs isomères ainsi que le cas échéant, leurs sels d'addition à un acide ou une base pharmaceutiquement acceptables.

6. Procédé de préparation selon la revendication 1 de composés pour lesquels A-B représentent un atome d'hydrogène, leurs isomères ainsi que le cas échéant, leurs sels d'addition à un acide ou une base pharmaceutiquement acceptable.

7. Procédé de préparation selon la revendication 1 de composés de formule générale (I/F) :

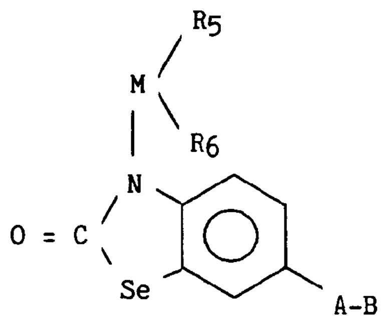

(I/F)

pour lesquels M représente un chaînon alkyle inférieur et A, B, $R_5$ et $R_5$ ont la même définition que dans la formule (I), leurs isomères ainsi que leurs sels d'addition à un acide ou une base pharmaceutiquement acceptables.

**8.** Procédé de préparation selon la revendication 1 de composés de formule (I/S) :

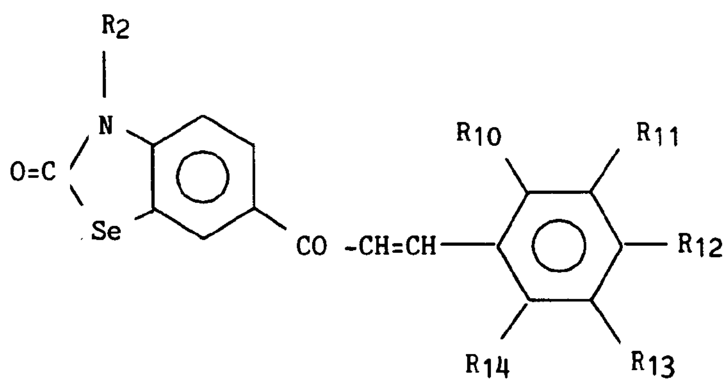

(I/S)

dans laquelle $R_2$ a la même signification que dans la revendication 1 et $R_{10}$, $R_{11}$, $R_{12}$, $R_{13}$ et $R_{14}$ identiques ou différents représentent indépendamment l'un de l'autre un atome d'hydrogène, un groupement alkyle inférieur, alkoxy inférieur, hydroxy, trifluorométhyle, amino ou atome d'halogène, leurs isomères ainsi que le cas échéant, leurs sels d'addition à un acide ou une base pharmaceutiquement acceptable.

**9.** Procédé de préparation selon la revendication 1 de composés pour lesquels A représente un groupement CO et B représente un groupement phényle, leurs isomères ainsi que le cas échéant, leurs sels d'addition à une base pharmaceutiquement acceptable.

**10.** Procédé de préparation selon la revendication 1 du composé qui est la 6-(3',5'-ditert.butyl 4'-hydroxy)cinnamoyl benzosélénazolinone ses isomères, ainsi que ses sels d'addition à une base pharmaceutiquement acceptable.

**11.** Procédé de préparation selon la revendication 1 du composé qui est la 3-méthyl 6-(3',5'-ditertbutyl 4'-hydroxy)cinnamoyl benzoselenazolinone, ses isomères, ainsi que ses sels d'addition à une base pharmaceutiquement acceptable.

**12.** Procédé de préparation selon la revendication 1 du composé qui est la 6-benzoyl benzosélénazolinone ainsi que ses sels d'addition à une base pharmaceutiquement acceptable.

**13.** Procédé de préparation selon la revendication 1 du composé qui est le bis [(2-amino 5-benzoyl) phényl] diséléniure ainsi que ses sels d'addition à un acide pharmaceutiquement acceptable.

**14.** Procédé de préparation selon la revendication 1 du composé qui est le bis [(2-méthyl amino 5-benzoyl) phényl] diséléniure ainsi que ses sels d'addition à un acide pharmaceutiquement acceptable.

**15.** Procédé de préparation selon la revendication 1 du composé qui est le bis [2-(3,3-diméthyl uréido) phényl] diséléniure.

**16.** Procédé de préparation selon la revendication 1 du composé qui est le bis [2-amino 5-(phényl hydroxy méthyl)phényl] diséléniure ainsi que ses sels d'addition à une base pharmaceutiquement acceptable.

**17.** Procédé de préparation selon la revendication 1 du composé qui est le bis [2-(3,3-diméthyl uréido)5-benzoyl phényl] diséléniure.

**18.** Procédé de préparation selon la revendication 1 du composé qui est le bis [2-amino 5-acétyl phényl] diséléniure ainsi que ses sels d'addition à un acide pharmaceutiquement acceptable.

**19.** Procédé de préparation selon la revendication 1 du composé qui est le bis [(2-méthyl amino) phényl]diséléniure ainsi que ses sels d'addition à un acide pharmaceutiquement acceptable.

**20.** Procédé de préparation selon la revendication 1 du composé qui est la 6-[(phényl) hydroxy méthyl] benzosélénazolinone ainsi que ses sels d'addition à une base pharmaceutiquement acceptable.

**21.** Procédé de préparation de composition pharmaceutique contenant comme principe actif au moins un composé obtenu selon l'une des revendications 1 à 20 en combinaison avec un ou plusieurs excipients ou véhicules inertes, non toxiques, pharmaceutiquement acceptables.

**22.** Procédé de préparation de composition pharmaceutique selon la revendication 21 contenant au moins un principe actif obtenu selon l'une des revendications 1 à 20 utilisables dans le traitement des troubles inflammatoires et plus particulièrement dans les troubles rhumatismaux, les hyperlipidémies, le diabète non insulino dépendant et ses complications, dans la prévention et le traitement des accidents ischémiques artériels périphériques et cérébro vasculaires, dans le traitement de certains cancers ainsi qu'en tant que conservateur de certains organes.

Office européen des brevets

# RAPPORT DE RECHERCHE EUROPEENNE

Numero de la demande

EP 91 40 3146

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 395 526 (ADIR ET COMPAGNIE)<br>* page 2; revendications * | 1,22,23 | C07D293/12<br>C07C391/02<br>A61K31/41<br>A61K31/095<br>C07D421/06 |
| D,A | CHEMICAL ABSTRACTS, vol. 30, no. 6, 20 Mars 1936, Columbus, Ohio, US;<br>abstract no. 1787-5,<br>R.F.HUNTER: 'Unsaturation and tautomeric mobility of heterocyclic compounds VI The mobility of 5-substituted 1-hydroxybenzothiazoles and the ultraviolet absorption of mobile and static derivatives of 1-hydroxy benzothiazole'<br>* abrégé *<br>& J.CHEM SOC<br>1935,<br>pages 1755 - 1761; | 1,2 | |
| A | CHEMICAL ABSTRACTS, vol. 92, no. 15, 14 Avril 1980, Columbus, Ohio, US;<br>abstract no. 128024C,<br>B.KH. STRELETS: 'Hydrolysis of benzo-1,2,3-dithiazolium salts and their selenium analogs'<br>page 619 ;<br>* abrégé *<br>& KHIM.GETEROTSIKL. SOEDIN<br>no. 9, 1979,<br>pages 1205 - 1210; | 1,3 | DOMAINES TECHNIQUES RECHERCHES (Int. Cl.5)<br>C07D<br>C07C |
| A | CHEMICAL ABSTRACTS, vol. 103, no. 11, 16 Septembre 1985, Columbus, Ohio, US;<br>abstract no. 87883G,<br>page 611 ;<br>* abrégé *<br>& CS-A-217 354 (J.PRACHAR ET AL) 1 Septembre 1984 | 1,3 | |

Le présent rapport a été établi pour toutes les revendications

| Lieu de la recherche | Date d'achèvement de la recherche | Examinateur |
|---|---|---|
| LA HAYE | 12 FEVRIER 1992 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)